# EUROPEAN PATENT APPLICATION

(11) **EP 4 430 948 A1**
(43) Date of publication of application: **18.09.2024**
(21) Application number: 23162583.1
(22) Date of filing: 17.03.2023
(51) Int. Cl.: A01N 63/20, A01N 63/22, A01N 63/27, B09C 1/10, C12N 1/20

(54) **STROBILURIN DEGRADING BACTERIA AND COMPOSITIONS**

(71) Applicant: Multikraft Produktions- und Handels GmbH, 4632 Pichl/Wels (AT)
(72) Inventor: Kraxberger, Katharina, 4632 Pichl/Wels (AT)
(74) Representative: Grünecker Patent- und Rechtsanwälte PartG mbB

(57) **Abstract**

The present invention relates to the degradation of strobilurin using plant-associated microorganisms, methods of reducing the strobilurin content of or on plants and/or soil, especially crop plants and compositions comprising strobilurin degrading plant-associated microorganisms.

## Description

### Field of the Invention

The present invention relates to the degradation of strobilurin using plant-associated microorganisms, methods of reducing the strobilurin content of plants and/or soil, especially crop plants and compositions comprising strobilurin degrading plant-associated microorganisms.

### Introduction

Currently, over 500 pesticides are registered and used worldwide in agriculture. Due to spray drifts, surface runoff and leaching processes, most of the applied pesticides (70-80%) inevitably disseminate and leave target sites, contaminating soils and water and pose a potential risk for humans and other life forms. Physical treatments like adsorption or percolator filters, as well as chemical treatments such as advanced oxidation or photodegradation for bioremediation of polluted sites, are cost-ineffective, monitoring-intensive and incomplete due to the generation of more persistent or/and more toxic metabolites and often only operate as *ex situ* clean-up technologies.

Fungicidal active substances are regularly detected in different vegetables and fruits. About 25% of the fungicides sold worldwide belong to the class of β-methoxyacrylate and the strobilurin family.

Only a limited number of azoxystrobin-degrading bacteria has been isolated so far. Isolated strains include members of the genera *Cupriavidus, Rhodanobacter* (Howell et al., 2014, Chemosphere 95, 370-378), *Bacillus* (Baćmaga *et al.,* 2017, Water, Air Soil Pollut. 228, 1-9) and *Ochrobactrum* (Feng et al., 2020, Microorganisms 8, 1-17). These strains were isolated from pesticide contaminated soils (Baćmaga *et al.,* supra; Howell *et al.,* supra) or sludge from wastewater treatment systems (Feng *et al.,* supra), but not from plants.

Therefore, there remains a need for methods and microbial compositions that can be used to degrade strobilurin fungicides on the surface of plants, in the plants themselves and in the soil, wherein preferably these plants are crop plants, as well as further bioremediation methods for polluted soils without the negative consequences of expensive and ineffective in situ bioremediation procedures. A further advantage of the methods and microbial compositions of the present invention is that they can be applied in organic agriculture for the production of organic fruit and vegetables so that they meet the strict regulatory requirements for organic food production. The methods and microbial compositions of the invention therefore help to meet the growing demand of food production and in particular organic food production. The strobilurin-degrading, plant-associated bacteria of the present invention can reduce strobilurin residue levels (e.g. azoxystrobin) in lettuce and other crops and represent a source of valuable strains to be applied for reducing strobilurin (e.g. azoxystrobin) contamination in plant products and soils used for crop plant production.

### Summary of the Invention

The invention relates in a fist aspect to a method for reducing the content of a strobilurin or a strobilurin derivative on a plant surface, in a plant or in soil, comprising (i) applying at least one bacterium selected from the group consisting of *Bacillus subtilis* MK101 with the accession no. DSM34510, *Rhodococcus fascians* MK144 with the accession no. DSM34512, *and Pseudomonas kermanshahensis* MK113 with the accession no. DSM34511, or a bacterium with an 16S rRNA sequence having at least 97%, 98%, or 99% sequence identity with the 16S rRNA sequence of *Bacillus subtilis* MK101 of SEQ ID NO:1, *Rhodococcus fascians* MK144 of SEQ ID NO:2, *or Pseudomonas kermanshahensis* MK113 of SEQ ID NO:3, or any combination of these bacteria, to at least one aerial part of the plant and/or to the soil, and (ii) incubating the plant and/or the soil for a time sufficient to reduce the content of the strobilurin on the plant surface and/or in the plant and/or in the soil. In a preferred embodiment the at least one bacterium or the combination of the bacteria is applied alone, or in conjunction with other/further microorganisms. In a preferred embodiment the at least one bacterium is selected from the group consisting of *Bacillus subtilis* MK101, *Rhodococcus fascians* MK144, and *Pseudomonas kermanshahensis* MK113, or a combination of any of these bacteria is applied alone, or in conjunction with other microorganisms. Particularly preferred are methods wherein at least one, or two or all three of *Bacillus subtilis* MK101 with the accession no. DSM34510, *Rhodococcus fascians* MK144 with the accession no. DSM34512, and *Pseudomonas kermanshahensis* MK113 with the accession no. DSM34511 is applied. The methods may include further steps, such as the application of bioorganic fertilizers and/or the application of compositions or microbial compositions that treat of prevent plant diseases and or plant pathogens, and/or compositions or microbial compositions for maintaining or improving the plant health.

In the methods described herein the at least one bacterium or the combination of bacteria can be applied in form of a composition, and preferably the composition can be applied as a foliar. Foliar compositions are well known in the art (Preininger et al. Applied Microbiology and Biotechnology (2018) 102:7265-7282)

In the methods described herein the concentration of the at least one bacterium which is applied is not particularly limited and may range from about 1 × 10⁵ to about 1 × 10⁷ cfu per plant per application, or from about 5 × 10⁵ to about 5 × 10⁷ cfu per m² of soil per application.

A further aspect of the invention relates to the use of at least one bacterium selected from the group consisting of *Bacillus subtilis* MK101 with the accession no. DSM34510, *Rhodococcus fascians* MK144 with the accession no. DSM34512, *and Pseudomonas kermanshahensis* MK113 with the accession no. DSM34511, or a bacterium with an 16S rRNA sequence having at least 97%, 98%, or 99% sequence identity with the 16S rRNA sequence of *Bacillus subtilis* MK101 of SEQ ID NO:1, *Rhodococcus fascians* MK144 of SEQ ID NO:2, *or Pseudomonas kermanshahensis* MK113 of SEQ ID NO:3, or any combination of these bacteria, for reducing the strobilurin content on a plant surface and/or in a plant and/or in soil, by applying said at least one bacterium or combination of bacteria to at least one aerial part of the plant and/or to the soil. In a preferred embodiment the at least one bacterium or the combination of the bacteria is applied alone, or in conjunction with other/further microorganisms. In a preferred embodiment the at least one bacterium is selected from the group consisting of *Bacillus subtilis* MK101, *Rhodococcus fascians* MK144, and *Pseudomonas kermanshahensis* MK113, or a combination of any of these bacteria is applied alone, or in conjunction with other microorganisms.

In one embodiment the at least one bacterium or the combination of bacteria is applied in form of a composition, preferably the composition can be applied as a foliar.

In any of the foregoing aspects the at least one bacterium or the combination of bacteria or the composition are applied to the leaves and/or the rhizosphere of the plant and most preferably the at least one bacterium or the combination of bacteria or the composition are applied to the leaves of the plant.

In any of the foregoing aspects the strobilurin is selected from the group consisting of Azoxystrobin, Dimoxystrobin, Fluoxastrobin, Kresoxim-methyl, Metominostrobin, Orysastrobin, Picoxystrobin, Pyraclostrobin, and Trifloxystrobin, particularly preferred is the degradation of Azoxystrobin.

In another aspect the invention relates to a composition comprising (a) at least one bacterium selected from the group consisting of *Bacillus subtilis* MK101 with the accession no. DSM34510, *Rhodococcus fascians* MK144 with the accession no. DSM34512, *and Pseudomonas kermanshahensis* MK113 with the accession no. DSM34511, alone or in combination with other microorganisms, or a bacterium with an 16S rRNA sequence having at least 97%, 98%, or 99% sequence identity with the 16S rRNA sequence of *Bacillus subtilis* MK101 of SEQ ID NO:1, *Rhodococcus fascians* MK144 of SEQ ID NO:2, or *Pseudomonas kermanshahensis* MK113 of SEQ ID NO:3, or any combination of these bacteria, optionally in further combination with other microorganisms and (b) at least one additive. Preferably, the composition is a foliar.

In any of the foregoing aspects the at least one bacterium does not express *strH* (such as GenBank Acc No. MT992707). In any of the foregoing aspects the at least one bacterium has genes for (i) benzoate degradation, preferably *catA, fadB* and/or *pcaC,* and/or (ii) aminobenzoate degradation, preferably *pdhC, ubiX* and/or *vanB* and/or (iii) genes encoding enzymes in the lactoylglutathione lyase family, preferably *cadI, gloA, gloA_2, mce, ydfO, yetH, ykcA, yodE, yqjT, yurT, ywbC, ywkD, yyaH* and/or *yycE.*

### Description of the Figures

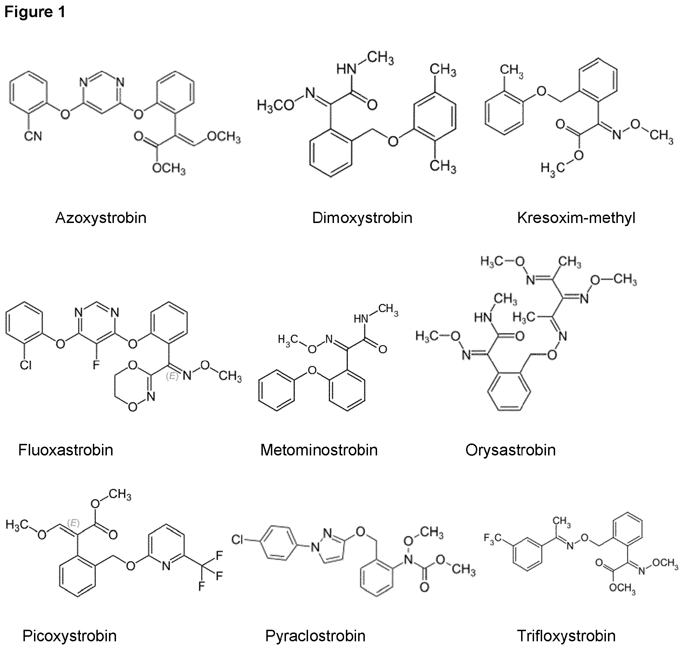

Figure 1: Selected strobilurin derivatives

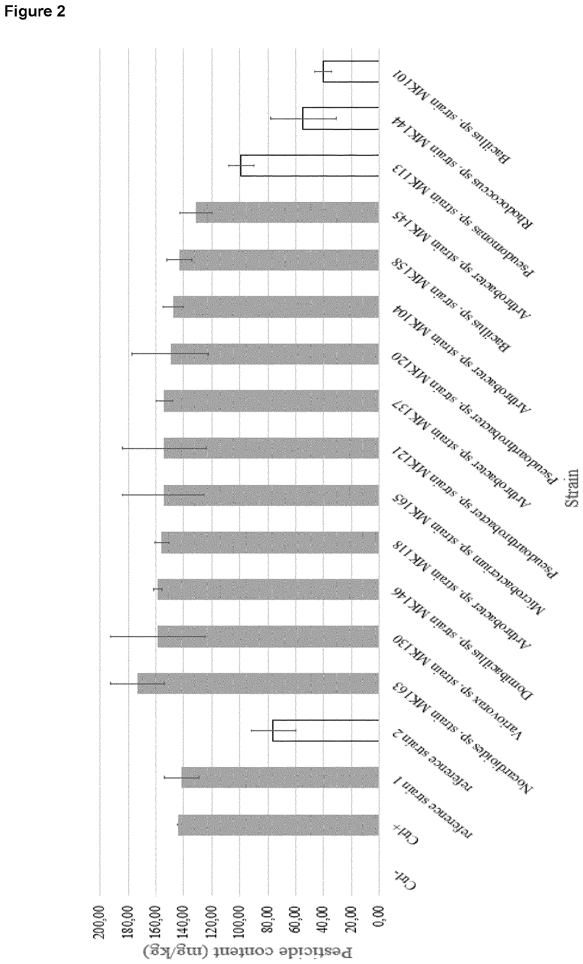

Figure 2: In vitro degradation of azoxystrobin; azoxystrobin content in the leaves (mg/kg) of the *in vitro* test (mean values of the triplicates ± SEM) after treatment with selected bacterial strains. The negative control are uninoculated lettuce samples without azoxystrobin treatment and the positive control are uninoculated lettuce samples with azoxystrobin treatment. Strains with filled bars showed no or only low degradation of azoxystrobin. Strains with empty bars showed degradation rates between 29% and 72% and were selected for greenhouse experiments. The bars from left to right are negative control (Ctrl -), positive control (Ctrl +), reference strain 1, reference strain 2, Nocardioides sp. strain MK163, Variovorax sp. strain MK130, Domibacillus sp. strain MK146, Arthrobacter sp. strain MK118, Microbacterium sp. strain MK165, Pseudoarthrobacter sp. strain MK121, Arthrobacter sp. strain MK137, Pseudoarthrobacter sp. strain MK120, Arthrobacter sp. strain MK104, Bacillus sp. strain MK158, Arthrobacter sp. strain MK145, Pseudomonas sp. strain MK113, Rhodococcus sp. strain MK144, Bacillus sp. strain MK101.

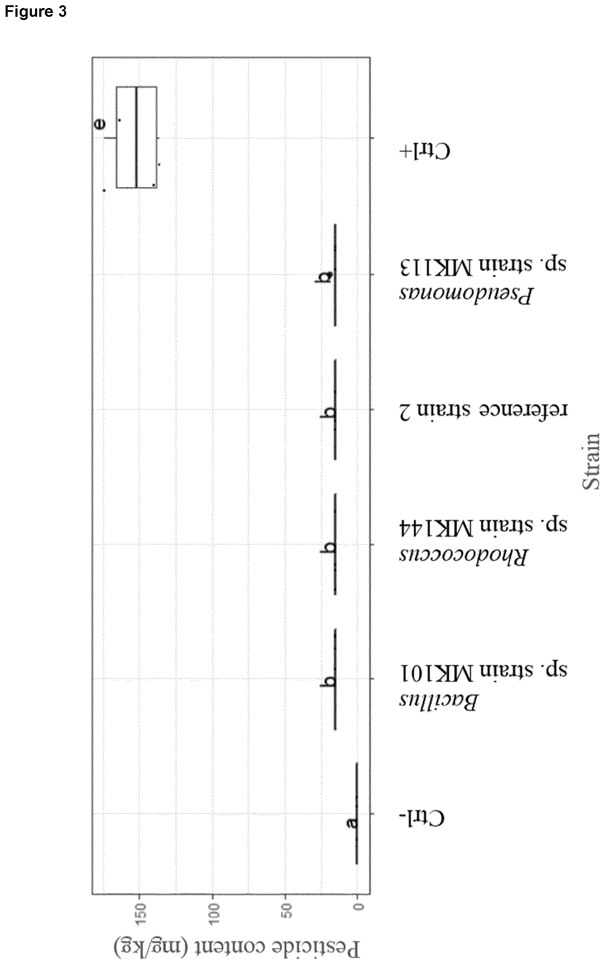

Figure 3: In planta degradation of azoxystrobin (foliar application of bacteria); azoxystrobin concentrations in the leaves (mg/kg) after spraying the fungicide and the different bacterial strains at the leaves (n=4). Dots represent the measured values. Lines within boxes represent median values. Different letters represent significant differences between measured pesticide contents (recursive partitioning analysis; p<0.005).

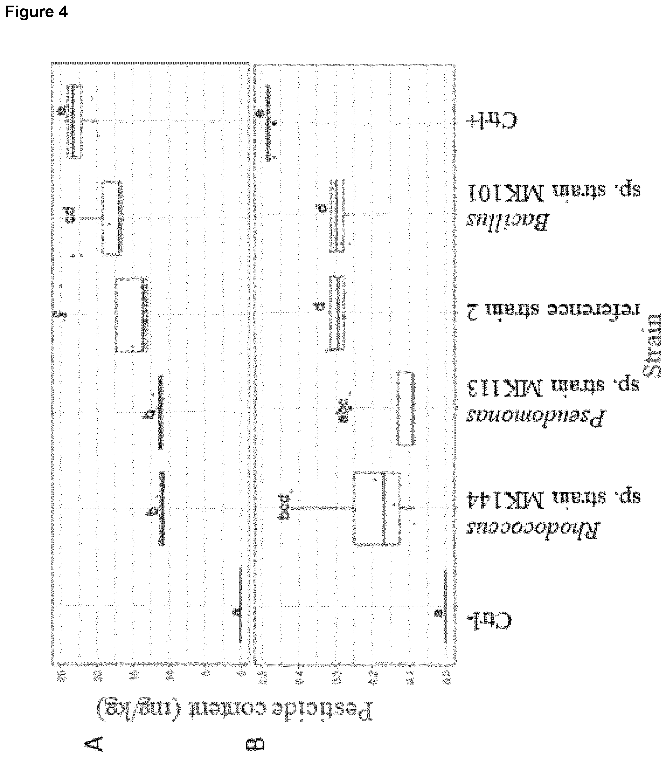

Figure 4: In planta degradation of azoxystrobin (soil application of bacteria); azoxystrobin concentrations in the soil (A) (n=8) and in the leaves (B) (n=4) after soil application and translocation to the shoot. Lines within boxes represent median values. Different letters represent significant differences between measured pesticide contents (recursive partitioning analysis; p<0.005).

### Detailed Description of the Invention

Unless defined otherwise, all technical and scientific terms used herein have the same meaning as commonly understood by those of ordinary skill in the art. Although any methods and materials similar or equivalent to those described herein can be used in the practice or testing of the present disclosure, typical methods and materials are described.

All publications, including but not limited to patents, patent applications and scientific publications, cited in this description are herein incorporated by reference for all purposes as if each individual publication were specifically and individually indicated to be incorporated by reference.

The use of the term "comprising" as well as other grammatical forms such as "comprises" and "comprised" is not limiting. The terms "comprising", "comprises" and "comprised" should be understood as referring to an open-ended description of an embodiment of the present invention that may, but does not have to, include additional technical features in addition to the explicitly stated technical features. In the same sense, the term "involving" as well as other respective grammatical forms such as "involves" and "involved" is not limiting. The same applies for the term "including" and other grammatical forms such as "includes" and "included". Section headings throughout the description are for organizational purposes only. In particular, they are not intended as limiting for various embodiments described therein, and it is to be understood that aspects, embodiments (and features therein) described under one subheading may be freely combined with aspects, embodiments (and features therein) described under another subheading. Further, the terms "comprising", "involving" and "including", and any grammatical forms thereof, are not to be interpreted to exclusively refer to embodiments that include additional features to those explicitly recited. These terms equally refer to embodiments that consist of only those features that are explicitly mentioned.

As used herein, the term "about" when referring to a measurable value such as an amount, a temporal duration, and the like, is meant to encompass, in addition to that measurable value itself, variations of ±20% or ±10%, in some instances ±9%, ±8%, ±7%, ±6%, ±5%, ±4%, ±3%, ±2% or ±1%, and in some instances ±0.9%,±0.8%, ±0.7%, ±0.6%, ±0.5%, ±0.4%, ±0.3%, ±0.2% or ±0.1% from the specified value. It is to be understood that the term "about", in reference to a particular value, includes that exact particular value itself, irrespective of any explicit mention that that exact particular value is included. Thus, the absence of an explicit indication that the term "about" includes the particular exact recited value is not to be understood that the particular recited value is excluded from the range of variations created by the term "about"; even in the absence of an explicit indication that the term "about" includes the particular exact recited value, that exact particular value is still included in said range of variations created by the term "about". The skilled person will recognize when deviations around a stated value may be either integral or fractional (as for example for a temperature or a pressure), and when such deviations may only be integral (as for example for discrete moieties within a larger molecule).

"Several" days refers to two to fourteen days, e.g., about 2, 3, 4, 5, 6, 7, 8, 9, 10, 11, 12, 13 or 14 days.

The term "effective amount" refers to an amount of bacteria or bacterial composition applied to a plant or a given area of soil or vegetation that is sufficient to effect one or more beneficial or desired outcomes, for example, fungicide, specifically strobilurin, reduction. An "effective amount" can be provided by administering the bacteria or bacterial composition once, twice, three times, four times, multiple times or intermittently. The duration between the administrations may be a few days, e.g., about 2, 3, 4, 5, 6, 7, 8, 9, 10, 11, 12, 13, 14, 15, 16, 17, 18, 19 or 20 days. The duration between the administrations may be a few weeks, e.g., about 2, 3, 4, 5, 6, 7, 8, 9, 10, 11, 12, 13, 14, 15, 16, 17, 18, 19 or 20 weeks.

A used herein the term "applying" in the context of the bacteria or bacterial compositions generally means bringing into contact with. Application of the bacteria or bacterial composition to a plant and/or a soil includes administration of the bacteria or bacterial composition to the plant or the soil or otherwise bringing the bacteria or bacterial composition into contact with the plant or the soil whether directly or indirectly. For example, exposing a plant to the bacteria or bacterial composition may include applying or administering the bacteria or bacterial composition to an environment inhabited by the plant or to a liquid or other nutrient composition to be administered to the plant. In the present disclosure the terms "applying", "administering" "exposing" and "contacting" and variations thereof may, in some contexts, be used interchangeably.

"Incubating" and similar terms like "incubation" and "incubate" means that the plant or soil that is incubated is left to rest for the time of the incubation, i.e. is not further processed or further actively treated. In this context, a time "sufficient" to reduce the content of strobilurin is a time that is long enough to allow the bacteria or the bacterial composition of the present invention to degrade, at least in parts, the strobilurin on or in the plant and/or soil. A skilled person can determine the time that is sufficient depending on the purpose or the intended use of the plant or soil. For example, if at a given time point the content of strobilurin of the plant is above the regulatory requirements that would allow the plant to be processed as food, a skilled person can determine the incubation time needed by way of measuring the strobilurin content of the plant at subsequent time points after application of the bacteria or the bacterial composition and thereby determine the rate at which the strobilurin is degraded or reduced by the current application scheme and thus determine the time that is sufficient to reach a concentration of strobilurin that indicates that the plant is suitable for food production.

The term "degrading" and variations thereof such as "degradation" and "degrade" as used herein in the context of pesticides, such as fungicides and in particular strobilurin (collectively referred to as substance to be degraded) refers to any activity of the bacteria or bacterial composition described herein that leads to a reduction of the content or amount of the relevant substance to be degraded on the surface of the plant or in the plant and/or on or in the soil in which the plant grows. Such reduction may be absolute or partial. The content or amount of the substance to be degraded on the surface of the plant (also referred to as on the plant) or in the plant and or soil by the inventive bacterial strain(s) or composition is by at least or about 5%, 10%, 15%, 20%, 25%, 30%, 35%, 40%, 45%, 50%, 55%, 60%, 65%, 70%, 75%, 80% 85%, 90%, 95% or more compared to the plant/soil that is not exposed to the bacterial strain(s) or composition.

The term "reducing" when referred to in the context of a fungicide such as strobilurin refers to the lowering of the amount of fungicide/strobilurin present in or on the plant or in the soil. The reduction in the amount may be a partial or a complete reduction and may refer to the absolute or relative content of the substance in or on the plant or in the soil. The terms "reducing" and "degrading" may in some contexts be used interchangeably.

The term "crop" as used herein refers to any plant grown to be harvested or used for any economic purpose, including for example human foods, livestock fodder, fuel or pharmaceutical production (e.g. poppies).

The term "identity" in the context of a %-identity of two biological sequences is used herein to describe the percentage of identical (not similar) amino acids or nucleotides of two sequences. A common method to determine the %-identity of two sequences is e.g. the BLAST algorithm (Needleman S.B. and Wunsch C.D. (1970), J. Mol. Biol. 48(3): 443-53) such as implemented in EMBOSS needle (Rice P., Longden I. and Bleasby A. (2000), EMBOSS: The European Molecular Biology Open Software Suite, Trends Genet. 16(6): 276-277) using the documented default configuration.

As used herein, the term "variant" or "variants" refers to both naturally occurring and specifically developed variants or mutants of the microbial strains disclosed and exemplified herein. Also encompassed by the term "variant" as used herein are microbial strains phylogenetically closely related to strains disclosed herein and strains possessing substantial sequence identity with the strains disclosed herein at one or more phylogenetically informative markers such as rRNA genes, elongation and initiation factor genes, RNA polymerase subunit genes, DNA gyrase genes, heat shock protein genes and recA genes. For example, the 16S rRNA genes of a "variant" strain as contemplated herein may share about 90%, 91%, 92%, 93%, 94%, 95%, 96%, 97%, 98% or 99% sequence identity with a strain disclosed herein, and preferably at least 97%. Sequences with greater than 97% identity are typically assigned to the same species (see Patrick D. Schloss and Jo Handelsman, Applied and Environmental Microbiology, Volume 71 Number 3, Pages: 1501-6, March 2005, and referenced cited therein). Accordingly, in one embodiment the composition of the invention comprises one or more variants of the disclosed bacterial species or strains. In particular, it is contemplated that the composition comprises one or more variants of the disclosed bacterial species or strains having at least about 90%, at least about 91%, at least about 92%, at least about 93%, at least about 94%, at least about 95%, at least about 96%, at least about 97%, at least about 98%, or at least about 99% identity in the 16S rRNA of the corresponding strain. For instance, in some embodiments, the microbial composition comprises one or more variants of any one of *Bacillus subtilis MK101 (DSM34510), Rhodococcus fascians MK144 (DSM34512),* and *Pseudomonas kermanshahensis MK113 (DSM34511)* having at least about 97%, 98% or 99% identity in the16S rRNA of the species. The 16S rRNA sequences of herein described strains are as follows:
>SEQ ID NO:1 - *Bacillus* sp. strain MK101 
TTCGGCGGCTGGCTCCTAAAAGGTTACCTCACCGACTTCGGGTGTTACAAACTCTCGTGGTGTGAC
GGGCGGTGTGTACAAGGCCCGGGAACGTATTCACCGCGGCATGCTGATCCGCGATTACTAGCGATT
CCAGCTTCACGCAGTCGAGTTGCAGACTGCGATCCGAACTGAGAACAGATTTGTGGGATTGGCTTAA
CCTCGCGGTTTCGCTGCCCTTTGTTCTGTCCATTGTAGCACGTGTGTAGCCCAGGTCATAAGGGGCA
TGATGATTTGACGTCATCCCCACCTTCCTCCGGTTTGTCACCGGCAGTCACCTTAGAGTGCCCAACT
GAATGCTGGCAACTAAGATCAAGGGTTGCGCTCGTTGCGGGACTTAACCCAACATCTCACGACACG
AGCTGACGACAACCATGCACCACCTGTCACTCTGCCCCCGAAGGGGACGTCCTATCTCTAGGATTG
TCAGAGGATGTCAAGACCTGGTAAGGTTCTTCGCGTTGCTTCGAATTAAACCACATGCTCCACCGCT
TGTGCGGGCCCCCGTCAATTCCTTTGAGTTTCAGTCTTGCGACCGTACTCCCCAGGCGGAGTGCTT
AATGCGTTAGCTGCAGCACTAAGGGGCGGAAACCCCCTAACACTTAGCACTCATCGTTTACGGCGT
GGACTACCAGGGTATCTAATCCTGTTCGCTCCCCACGCTTTCGCTCCTCAGCGTCAGTTACAGACCA
GAGAGTCGCCTTCGCCACTGGTGTTCCTCCACATCTCTACGCATTTCACCGCTACACGTGGAATTCC
ACTCTCCTCTTCTGCACTCAAGTTCCCCAGTTTCCAATGACCCTCCCCGGTTGAGCCGGGGGCTTTC
ACATCAGACTTAAGAAACCGCCTGCGAGCCCTTTACGCCCAATAATTCCGGACAACGCTTGCCACCT
ACGTATTACCGCGGCTGCTGGCACGTAGTTAGCCGTGGCTTTCTGGTTAGGTACCGTCAAGGTACC
GCCCTATTCGAACGGTACTTGTTCTTCCCTAACAACAGAGCTTTACGATCCGAAAACCTTCATCACTC
ACGCGGCGTTGCTCCGTCAGACTTTCGTCCATTGCGGAAGATTCCCTACTGCTGCCTCCCGTAGGA
GTCTGGGCCGTGTCTCAGTCCCAGTGTGGCCGATCACCCTCTCAGGTCGGCTACGCATCGTTGCCT
TGGTGAGCCGTTACCTCACCAACTAGCTAATGCGCCGCGGGTCCATCTGTAAGTGGTAGCCGAAGC
CACCTTTTATGTTTGAACCATGCGGTTCAAACAACCATCCGGTATTAGCCCCGGTTTCCCGGAGTTAT
CCCAGTCTTACAGGCAGGTTACCCACGTGTTACTCACCCGTCCGCCGCTAACATCAGGGAGCAAGC
TCCCATCTGTCCGCTCGACTTGCATGTATTAGGCACGCCGCCAGCGTTCGTCCTGAGCAG
>SEQ ID NO:2 - Rhodococcus sp. strain MK144 
GCGCTTAATGCGTTAGCTACGGCACAGATCCCGTGGAAGGAACCCACACCTAGCGCCCACCGTTTA
CGGCGTGGACTACCAGGGTATCTAATCCTGTTTGCTACCCACGCTTTCGTTCCTCAGCGTCAGTTGT
TTCCCAGAGACCCGCCTTCGCCACCGGTGTTCCTCCTGATATCTGCGCATTTCACCGCTACACCAGG
AATTCCAGTCTCCCCTGAAACACTCAAGTCTGCCCGTATCGCCTGCAAGCCCGAAGTTGAGCCCCG
GGTTTTCACAAACGACGCGACAAACCGCCTACGAACTCTTTACGCCCAGTAATTCCGGACAACGCTC
GCACCCTACGTATTACCGCGGCTGCTGGCACGTAGTTAGCCGGTGCTTCTTCTGCAGGTACCGTCA
CTCACGCTTCGTCCCTGCTGAAAGAGGTTTACAACCCGAAGGCCTTCATCCCTCACGCGGCGTCGC
TGCATCAGGCTTCCGCCCATTGTGCAATATTCCCCACTGCTGCCTCCCGTAGGAGTCTGGGCCGTG
TCTCAGTCCCAGTGTGGCCGGTCACCCTCTCAGGTCGGCTACCCGTCGTCGCCTTGGTAGGCCATT
ACCCCACCAACAAGCTGATAGGCCGCGGGCCCATCCTGCACCGATAAATCTTTCCACCACAACACAT
GCATGCTGTGGTCATATCCGGTATTAGACCCAGTTTCCCAAGCTTATCCCAGAGTGCAGGGCAGATC
ACCCACGTGTTACTCACCCGTTCGCCGCTCGTGTACCCCGAAAGGCCTTACCGCTCGACTTGCATG
TGTTAAGCACGCCGCCAGCGTTCGTCCTGAGCA
>SEQ ID NO:3 - Pseudomonas sp. strain MK113

CCGTCCTCCCGAAGGTTAGACTAGCTACTTCTGGTGCAACCCACTCCCATGGTGTGACGGGCGGTG
TGTACAAGGCCCGGGAACGTATTCACCGCGACATTCTGATTCGCGATTACTAGCGATTCCGACTTCA
CGCAGTCGAGTTGCAGACTGCGATCCGGACTACGATCGGTTTTGTGAGATTAGCTCCACCTCGCGG
CTTGGCAACCCTCTGTACCGACCATTGTAGCACGTGTGTAGCCCAGGCCGTAAGGGCCATGATGAC
TTGACGTCATCCCCACCTTCCTCCGGTTTGTCACCGGCAGTCTCCTTAGAGTGCCCACCATAACGTG
CTGGTAACTAAGGACAAGGGTTGCGCTCGTTACGGGACTTAACCCAACATCTCACGACACGAGCTG
ACGACAGCCATGCAGCACCTGTGTCAGAGTTCCCGAAGGCACCAATCCATCTCTGGAAAGTTCTCTG
CATGTCAAGGCCTGGTAAGGTTCTTCGCGTTGCTTCGAATTAAACCACATGCTCCACCGCTTGTGCG
GGCCCCCGTCAATTCATTTGAGTTTTAACCTTGCGGCCGTACTCCCCAGGCGGTCAACTTAATGCGT
TAGCTGCGCCACTAAAATCTCAAGGATTCCAACGGCTAGTTGACATCGTTTACGGCGTGGACTACCA
GGGTATCTAATCCTGTTTGCTCCCCACGCTTTCGCACCTCAGTGTCAGTATCAGTCCAGGTGGTCGC
CTTCGCCACTGGTGTTCCTTCCTATATCTACGCATTTCACCGCTACACAGGAAATTCCACCACCCTCT
ACCGTACTCTAGCTTGCCAGTTTTGGATGCAGTTCCCAGGTTGAGCCCGGGGCTTTCACATCCAACT
TAACAAACCACCTACGCGCGCTTTACGCCCAGTAATTCCGATTAACGCTTGCACCCTCTGTATTACC
GCGGCTGCTGGCACAGAGTTAGCCGGTGCTTATTCTGTCGGTAACGTCAAAACAGCAAGGTATTCG
CTTACTGCCCTTCCTCCCAACTTAAAGTGCTTTACAATCCGAAGACCTTCTTCACACACGCGGCATG
GCTGGATCAGGCTTTCGCCCATTGTCCAATATTCCCCACTGCTGCCTCCCGTAGGAGTCTGGACCGT
GTCTCAGTTCCAGTGTGACTGATCATCCTCTCAGACCAGTTACGGATCGTCGCCTTGGTGAGCCATT
ACCCCACCAACTAGCTAATCCGACCTAGGCTCATCTGATAGCGCAAGGCCCGAAGGTCCCCTGCTT
TCTCCCGTAGGACGTATGCGGTATTAGCGTTCCTTTCGAAACGTTGTCCCCCACTACCAGGCAGATT
CCTAGGCATTACTCACCCGTCCGCCGCTGAATCGAAGAGCAAGCTCTTCTCATCCGCTCGACTTGCA
TGTGTTAGGCCTGCCGCCAGCGTTCAATCTGAGCCTCGACTTGCATGTGTAAGGCATGCCGCCAGC
GTTCAATCTGAGCAG

The concentrations of each bacterial strain to be added to bacterial compositions as disclosed herein will depend on a variety of factors including the identity and number of individual strains employed, the plant species being treated, the nature and condition of the soil to be treated, the exact nature of the bacterial composition to be applied, the form in which the composition is applied and the means by which it is applied, and the stage of the plant growing season during which application takes place. For any given case, appropriate concentrations may be determined by one of ordinary skill in the art using only routine experimentation. Concentrations can be given as colony forming units (cfu or CFU) per Millilitre (ml). By way of example, the concentration of each strain in a bacterial composition may be from about 1 × 10² cfu/ml to about 1 × 10¹⁰ cfu/ml, and may be about 1 × 10³ cfu/ml, about 2.5 × 10³ cfu/ml, about 5 × 10³ cfu/ml, 1 × 10⁴ cfu/ml, about 2.5 × 10⁴ cfu/ml, about 5 × 10⁴ cfu/ml, 1 × 10⁵ cfu/ml, about 2.5 × 10⁵ cfu/ml, about 5 × 10⁵ cfu/ml, 1 × 10⁶ cfu/ml, about 2.5 × 10⁶ cfu/ml, about 5 × 10⁶ cfu/ml, 1 × 10⁷ cfu/ml, about 2.5 × 10⁷ cfu/ml, about 5 × 10⁷ cfu/ml, 1 × 10⁸ cfu/ml, about 2.5 × 10⁸ cfu/ml, about 5 × 10⁸ cfu/ml, 1 × 10⁹ cfu/ml, about 2.5 × 10⁹ cfu/ml, or about 5 × 10⁹ cfu/ml. The final concentration of the strains in the bacterial composition may preferably be between about 1 × 10⁴ cfu/ml and 1 × 10⁶ cfu/ml for each species or genus. The final concentration in the inventive bacterial composition may therefore range between from about 5 × 10³ to about 2.5 × 10¹⁰ cfu/ml and preferably is 1 × 10⁵, 2 × 10⁵, 3 × 10⁵, 4 × 10⁵, 5 × 10⁵ cfu/ml, ..., 6 × 10⁷, 7 × 10⁷, 8 × 10⁷, 9 × 10⁷ cfu/ml or higher.

For example 0,5-10 mL (e.g. 2, 3, or 4 mL) of a bacterial suspension (about 10⁶ cfu/mL) per plant and treatment may applied on two or more different time points after the fungicide treatment, e.g. after one or several days or weeks for the first time, and after one or several days or weeks after the first application. A skilled person will understand that the volume and CFU per plant (or per m² soil) as well as the number of applications will depend on the plant to be treated and/or the amount of strobilurin or strobilurin derivative to be degraded.

Compositions of the present disclosure may optionally further comprise one or more additional microbial organisms, for example, additional agronomically beneficial microorganisms. Such agronomically beneficial microorganisms may act in synergy or concert with, or otherwise cooperate with the organisms of the present disclosure in the composition. Examples of further agronomically beneficial microorganisms include *Bacillus* sp., *Pseudomonas* sp., *Rhizobium* sp., *Azospirillum* sp., *Azotobactersp.,* phototrophic and cellulose degrading bacteria, *Clostridium* sp., *Trichoderma* sp. and the like.

The crop plant may be, for example, a food crop (for humans or other animals) such as any fruit, vegetable, nut, seed or grain producing plant. Exemplary crop plants include, but are not limited to, tubers and other below-ground vegetables (such as potatoes, beetroots, radishes, carrots, onions, etc.), ground-growing or vine vegetables (such as pumpkin and other members of the squash family, beans, peas, asparagus, etc.), leaf vegetables (such as lettuces, chard, spinach, alfalfa, etc.), other vegetables (such as tomatoes, brassica including broccoli, avocadoes, etc.), fruits (such as berries, olives, stone fruits including nectarines and peaches, tropical fruits including mangoes and bananas, apples, pears, mandarins, oranges, mandarins, kiwi fruit, coconut, etc.), cereals (such as rice, maize, wheat, barley, millet, oats, rye etc.), nuts (such as macadamia nuts, peanuts, brazil nuts, hazel nuts, walnuts, almonds, etc.), and other economically valuable crops and plants (such as sugar cane, soybeans, sunflower, canola, sorghum, pastures, turf grass, etc.). Preferred plants are plants, wherein the harvestable parts are aboveground, and in particular leaf vegetables. For example, lettuce was shown to be a crop containing relatively high amounts of pesticide residues, including azoxystrobin.

The bacterial strain(s) or combination of strains or the compositions of the present disclosure may be applied directly to plants, plant parts (such as foliage), or alternatively may be applied to soil in which the plants are growing or are to be grown. Application may be by any suitable means and may be on any suitable scale. For example, application may comprise pouring, spreading or spraying, including broad scale or bulk spreading or spraying. Multiple means of application may be used in combination (for example application as a foliar to the plants and application to the soil by way of drop watering or drip irrigation). Seedlings or mature plants may be treated as many times as appropriate. The number of applications required can readily be determined by those skilled in the art, depending on, for example, the plant in question, the stage of development of the plant at which treatment is initiated, the state of health of the plant, the growth, environmental and/or climatic conditions in which the plant is grown and the purpose for which the plant is grown. For example, in the case of flowering crops such as tomatoes, it may be desirable to apply the bacterial strain(s) or combination of strains or the composition once or more than once during the flowering period.

The bacterial compositions as disclosed herein may be prepared in any suitable form depending on the means by which the composition is to be applied to the soil or vegetation. Suitable forms can include, for example, slurries, liquids, and solid forms. Solid forms are preferred for the use of soil bioremediation and include powders, granules, larger particulate forms and pellets (for example dyed or undyed gelatine spheres or capsules). Liquids are preferred for strobilurin degradation in or on plants and may include aqueous solutions and aqueous suspensions, and emulsifiable concentrates. However, liquid compositions can be used for soil bioremediation as well and may in particular be used for conjoint treatment of the plants and the soil in which the plants grow.

In order to achieve effective dispersion, adhesion and/or conservation or stability within the environment of the bacterial compositions disclosed herein, it may be advantageous to formulate the compositions with suitable carrier components that aid dispersion, adhesion and/or conservation/stability. Suitable carriers for the use of soil bioremediation will be known to those skilled in the art and include, for example, ceramics or ceramic powder, chitosan, vermiculite, compost, talc, chalk, carbon particles or powder, charcoal, rice hulls, gels and the like.

In one particular embodiment, the bacterial composition may take the form of a foliar. Essentially, a "foliar" is characterized in that it is in a form that is suitable for the application to plants by way of spraying the foliar composition directly on the aboveground parts of a plant. The foliar composition has a form that is suitable for wetting or (temporarily) covering and/or at least temporarily adhering to the surfaces to which it is applied, such that at least a fraction of the bacteria contained in the foliar composition are allowed to reside on and remain in contact with the treated plant. A foliar may contain one or more of the group selected from a surfactant and/or wetter (such as Polyethylenglycol, rhamnolipid, polyethyleneoxide, Tween 20), a sticker/adhesive (such as gelatin, gums, CMC, starch, PVA, PVP), a thickener (such as xanthan, CMC, agar, guar gum), a humectant (such as sorbitol, molasses, glycerol, sucrose, polyethylenglycols), a defoaming agent (such as fatty acids esters, oils, wax, polypropylene glycol), a stabilizer (such as milk powder, molasses, sucrose, trehalose), and an UV protectant (such as congo red, lignin, p-aminobenzoic acid, molasses, talc, nanoparticles). Methods for producing foliar spray applications are well known in the art (Preininger et al. Applied Microbiology and Biotechnology (2018) 102:7265-7282; herein incorporated by reference).

Additional components may be incorporated into compositions of the present disclosure, such as humic substances, trace elements, organic material, penetrants, macronutrients, micronutrients and other soil and/or plant additives. Humus or humic substances that may be incorporated may include, but are not limited to, humic acid derived from, for example oxidised lignite or leonardite, fulvic acid and humates such as potassium humate. Organic material added may include, but is not limited to, biosolids, animal manure, compost or composted organic by-products, activated sludge or processed animal or vegetable by-products (including blood meal, feather meal, cottonseed meal, ocean kelp meal, seaweed extract, fish emulsions and fish meal). Penetrants include, but are not limited to, non-ionic wetting agents, detergent based surfactants, silicones, and/or organo-silicones. Suitable penetrants will be known to those skilled in the art, non-limiting examples including polymeric polyoxyalkylenes, allinol, nonoxynol, octoxynol, oxycastrol, TRITON, TWEEN, Sylgard 309, Silwet L-77, and Herbex (silicone / surfactant blend).

The method for reducing the content of a strobilurin or a strobilurin derivative on a plant surface, in a plant or in soil, comprises at least (i) the step of applying at least one bacterium selected from the group consisting of *Bacillus subtilis* MK101 with the accession no. DSM34510, *Rhodococcus fascians* MK144 with the accession no. DSM34512, *Pseudomonas kermanshahensis* MK113 with the accession no. DSM34511, or a bacterium with an 16S rRNA sequence having at least 97%, 98%, or 99% sequence identity with the 16S rRNA sequence of *Bacillus subtilis* MK101 of SEQ ID NO:1, *Rhodococcus fascians* MK144 of SEQ ID NO:2, *Pseudomonas kermanshahensis* MK113 of SEQ ID NO:3, or any combination of these bacteria, to at least one aerial part of the plant and/or to the soil, and (ii) a step of incubating the plant and/or the soil for a time sufficient to reduce the content of the strobilurin on the plant surface and/or in the plant and/or in the soil.

For the methods of the present invention, the bacterial composition may be formulated in any appropriate form for the application to plants or as soil treatment and can be applied at any appropriate time, or in any appropriate time intervals depending on the use case. Typically, for the treatment of plants growing on land plots about 0.1 to about 50 L, preferably about 0.5 to about 25 Land in particular about 1-5 to about 10-20 L per ha of a bacterial composition containing a CFU/ml as described above may be used. For soil treatments about 0.3-0.5 to about 300 L, preferably about 1 to about 150 Land in particular about 2-10 to about 40-60 L per ha of a bacterial composition containing a CFU/ml as described above may be used. If used in combination with a growth enhancer, the bacterial compositions may typically be applied a plurality of times before and/or during the growth period of the plants, depending on the soil type and its agricultural history (previous agricultural use, previous fertilizing regime, etc.). The application may occur once before and/or after planting and/or once or several times weekly/monthly. For acute strobilurin decontamination treatment, the bacterial compositions may be used once, or a plurality of times until the strobilurin content is reduced or until time for harvest of the plants.

The strain *Bacillus subtilis* MK101 was deposited with the Leibnitz-lnstitut DSMZ-Deutsche Sammlung von Mikroorganismen und Zellkulturen (DSMZ) as international depositary authority in line with the Budapest treaty by the applicant on 2023-01-25 and was given the Accession no. DSM34510, *Rhodococcus fascians* MK144 was deposited with the DSMZ by the applicant on 2023-01-25 and was given the Accession no. DSM34512, *Pseudomonas kermanshahensis* MK113 was deposited with the DSMZ by the applicant on 2023-01-25 and was given the Accession no. DSM34511. The present invention relates to these strains as well as to derivatives of these strains.

"Derivative strains" comprise deposited strains that have been modified by transformation with vectors of any kind, or deposited strains that have been genetically modified by any technique for modifying bacterial genomes such as gene gun, gene editing (e.g. CRISPR/Cas9), viral transduction, random mutagenesis, triparental conjugation with related and/or unrelated strains, transduction, transfection, electroporation, gene insertion, mutagenesis, phage infection, conjugation, protoplast fusion, modification by gene deletion, gene replacement or gene insertion. A derivative strain of a deposited strain comprises any bacterium of of *Bacillus subtilis* MK101 with the accession no. DSM34510, *Rhodococcus fascians* MK144 with the accession no. DSM34512, or *Pseudomonas kermanshahensis* MK113 with the accession no. DSM34511 that contains a nucleic acid that is heterologous to the strain, or contains an additional copy of an endogenous nucleic acid (gene duplication, artificially increased copy number), or has a endogenous rearrangement of genes (gene translocation, change in the order of genes on the genome) relative to the deposited strain. The skilled person will be able to determine the genomic sequence of the inventive strains based on the deposits and be able to produce and recognize derivative strains.

Strobilurin is a class of fungicides with the generic formula (I): 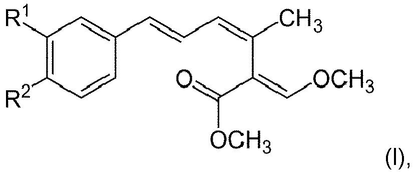 wherein R¹ is selected from the group consisting of -H, -OCH₃, -OCH₂-CH=C(CH₃)₂, and -OH and R² is selected from the group consisting of -H, -Cl and -OCH₂-CH=C(CH₃)₂. Strobilurin includes strobilurin A, 9-Methoxystrobilurin A, strobilurin B, C, D, G, E, F1, F2, H and X. For the present disclosure, the term "strobilurin" includes synthetic strobilurins and strobilurin derivatives of the aforementioned strobilurins as well as oudemansins of formula (II) 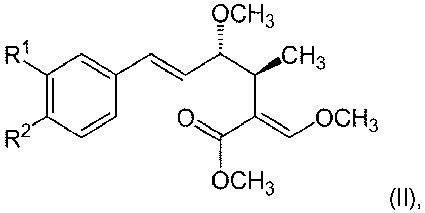 wherein R¹ is -H or -OCH₃ and R² is selected from the group consisting of -H, -Cl and -OCH₃. Oudemansin includes Oudemansin A, B and X.

Further fungicides of the strobilurin class that are encompassed by the term "strobilurin" in the context of the present invention are synthetic strobilurins such as azoxystrobin (CAS 131860-33-8), dimoxystrobin (CAS 149961-52-4), fluoxastrobin (CAS 361377-29-9), kresoxim-methyl (CAS 143390-89-0), metominostrobin (CAS 133408-50-1), orysastrobin (CAS 248593-16-0), picoxystrobin (CAS 117428-22-5), pyraclostrobin (CAS 175013-18-0), and trifloxystrobin (CAS 141517-21-7). A preferred strobilurin that is susceptible to the degradation by the bacteria and bacterial compositions of the present invention is azoxystrobin. Azoxystrobin belongs to the class of β-methoxyacrylate and strobilurin family, which represent 25% of the fungicides sold worldwide.

The following examples demonstrate the efficacy of the microbial compositions of the present invention in the treatment and prevention of various plant infections caused by a number of plant pathogens. In addition, the examples show that the field application of the microbial compositions of the present invention exhibit a growth promoting effect on certain crop plants.

The bacterial compositions used in the Examples are exemplary compositions and shall not be construed to be limiting on the present invention.

### Examples

### Example 1. Isolation, de-replication and characterisation of fungicide-degrading strains

### Set-up of the greenhouse experiment and sampling of plants

Lettuce seeds (*Lactuca sativa L.,* Austrosaat AG, Austria) were sown in seed trays containing a mixture (3:1 v/v) of commercial potting soil (unit earth SP ED63T, Sinntal-Altengronau, Germany) and perlite (2-6 mm, GBC Gartenbaucentrum, Austria). After reaching the growth stage 13 of the BBCH scale, which is the third true leaf unfolded, the plants were trans-planted to 3 L pots (two plants per pot, six biological replicates, randomized layout). Pots were filled with azoxystrobin-contaminated soil, collected from a field in England-Sleaford (52°99'69.5"N; -°25'94.9"E) treated with the fungicide Azoxystar (200 g/L azoxystrobin, Life Scientific GmbH, Germany) for several years. Soil samples were collected from 20-25 cm depth and stored at 4°C prior to use. The temperature in the greenhouse was set to 22°C with a relative humidity of 30% during the day. The light value was kept between 10 klx and 40 klx by turning on artificial light or shading. During the night, the overall temperature was 20°C with 15% relative humidity.

Plants were watered with tap water twice a week from below using saucers to avoid fungicide washout. The pot arrangement, greenhouse settings and information about watering apply to all greenhouse experiments described in this application.

After ten days of growth, the number of plants per pot was reduced from two to one. After reaching the growth stage 49 of the BBCH scale, which is the typical size, form and firmness of lettuce heads, three replicates were randomly selected and carefully removed from the pot. Two compartments (leaves and rhizosphere) were sampled. The rhizosphere soil was removed from the root system by carefully shaking it off, whereas leaves were obtained by cutting the plants above the roots with a sterile scalpel. The tissue samples were cut into small pieces and mixed with sterile 0.9% NaCl (1:10 dilution) in a sterile pulsifierbag (PUL512 Pulsifier II^{®} Bags, Microgen Bioproducts Ltd, UK). These samples were used immediately for the isolation of bacteria.

### Isolation of bacteria from plants

Microbial cells were dislodged from plant tissue by using the Pulsifier (Pulsifier II^{®}, Microgen Bioproducts Ltd, UK) two times for 15 s. Dilutions of the supernatants were made with sterile 0.9% NaCl, and 100 µl of the selected dilutions, 10⁻¹ for the leaf and 10⁻³ for the rhizosphere samples, were plated in triplicates on diverse solid media containing fungicide as sole carbon source. Media were (i) 200 mL/L minimal medium M9 (33.9 g/L Na₂HPO₄, 15 g/L KH₂PO₄, 2.5 g/L NaCl, 5 g/L NH₄Cl) + 25 mg/L azoxystrobin dissolved in ethyl acetate (Azoxystrobin PESTANAL^{®}, Merck, Germany) + 15 g/L agar, (ii) 500 mL/L minimal salts medium MMS (0.1 g/L MgCl₂*6H₂O, 0.01 g/L FeCl₃*6H₂O, 0.005 g/L CaCl₂, 0.05 g/L NaCl, 0.5 g/L (NH₄)₂SO₄) + 25 mg/L azoxystrobin + 15 g/L agar, (iii) 200 mL/L minimal media M9 + 25 mg/L azoxystrobin + 30 g/L gellan gum, (iv) 500 mL/L minimal salts medium MMS + 25 mg/L azoxystrobin + 30 g/L gellan gum.

Plates were incubated for 72 h at 28°C. Ten and five morphologically different colonies from each of the four isolation media were selected from the plant multi-microbe products, respectively. Isolates were further cultivated on 200 mL/L minimal media M9 + 25 mg/L azoxystrobin + 15 g/L agar.

For gDNA extraction, a loop of cells grown on an agar plate was re-suspended in 200 µL sterile 0.9% NaCl, pelleted (18,407 rcf, 5 min) and stored at -20°C. gDNA of each isolate was extracted using the nexttec 1-Step DNA Isolation Kit for Bacteria (Biozym Scientific GmbH, Germany) according to the manufacturer's instructions.

### Characterisation and de-replication of isolates

Restriction fragment length polymorphism (RFLP) analysis of the 16S-23S rRNA intergenic spacer (IGS) region (IGS-RFLP-analysis) was performed to de-replicate the isolates and thus to identify unique bacterial strains. IGS PCR was performed with the primer pair P23SR01 (5'-GGCTGCTTCTAAGCCAAC-3', SEQ ID NO:4) and pHr (5'-TGCGGCTGGATCACCTCCTT-3', SEQ ID NO:5). A conventional PCR amplification in 50 µl PCR reaction mix containing 2.5 mM MgCl₂, 0.2 mM dNTPs, 0.3 mM of each primer, 4 µl DNA template, 1 U *Taq* DNA polymerase (Soils BioDyne, Estonia) and 1 × PCR reaction buffer was performed. An initial denaturation step at 95°C for 5 min was followed by 30 cycles of denaturation at 95°C for 45 s, annealing at 54°C for 60 s and elongation at 72°C for 120 s, followed by a final extension at 72°C for 10 min. Electrophoresis (100 V, 45 min) on 1% (w/v) agarose gels was used to test the quality of the fragments (expected amplicon size in the range 1,500 to 2,500 bp depending on the organism).

Restriction digest of IGS PCR products was performed using 200 ng amplified DNA, 5 U *Alu*I or Hhal endonuclease (FastDigest, Thermo Fisher Scientific, USA) and 1x Buffer Tango (Thermo Fisher Scientific, USA). Digestion was performed for 3 h at 37°C in a thermocycler. Electrophoresis (100 V, 120 min) on 2% (w/v) agarose gels was used to separate DNA fragments. RFLP patterns were compared (Reiter et al., 2002, Appl. Environ. Microbiol. 68, 2261-2268. https://doi.org/10.1128/AEM.68.5.2261-2268.2002) and isolates with identical IGS-RFLP patterns were excluded.

The 16S rRNA genes were amplified using the primers 8F (5'-AGAGTTTGATCCTGGCTCAG-3', SEQ ID NO:6) and 1520R (5'-AAGGAGGTGATCCAGCCGCA-3', SEQ ID NO:7). Conventional PCR amplification was performed, as described above, but in 25 µl PCR reaction mix and using 2 µl DNA template. The annealing temperature was set to 52°C. The expected amplicon size of the 16S rRNA amplicons is 1,500 bp.

Partial 16S rRNA sequencing was performed by LGC (LGC Group, UK) using the primer 926R (5'-CCGTCAATTCCTTTGAGTTT-3', SEQ ID NO:8) and 1492R (5'-GGTTACCTTGTTACGACTT-3', SEQ ID NO:9).

To obtain complete 16S rRNA gene sequences of selected strains, the ends of partial sequences were trimmed and assembled using Geneious Prime^{®} 2022.1.1. Decipher (Wright, 2016, Using DECIPHER v2.0 to Analyze Big Biological Sequence Data in R. R J. 8, 352-359. https://doi.org/10.32614/rj-2016-025) was used to check for chimeric sequences. The closest species match of assembled sequences was identified using Basic Local Alignment Search Tool (BLAST) analysis.

Results: In total, 44 different strains were obtained from lettuce plant isolates after excluding isolates with identical IGS-RFLP patterns. One *Arthrobacter* strain (*Arthrobacter* sp. strain MK97), one *Bacillus* strain (*Bacillus* sp. strain MK158) as well as one *Ensifer* strain (*Ensifer* sp. strain MK141) were obtained from rhizosphere and from leaf samples. Twenty-four different strains (belonging to 13 genera) were isolated from lettuce rhizosphere, whereas 22 strains (belonging to eleven genera) were obtained from leaves. Potential degraders from rhizosphere samples were bacteria from the genera *Ensifer, Neorhizobium, Rhizobium, Acidovorax, Pelomonas, Variovorax, Pseudomonas, Rhizobacter, Arthrobacter, Lentzea, Streptomyces, Bacillus* and *Flavobacterium.* Potential degraders from leaf samples were bacteria from the genera *Ensifer, Microbacterium, Arthrobacter, Pseudoarthrobacter, Rhodococcus, Nocardioides, Streptomyces, Bacillus, Domibacillus, Paenibacillus* and *Dyadobacter* (Table 1).

| **Strain number(s)¹** | **Closest 16S rRNA BLAST Hit (accession number)** | **% sequence identity** | **Isolation source** | **Degradation activity²** | |
|---|---|---|---|---|---|
| | | | | ***in vitro*** | ***in planta*** |
| | **Alphaproteobacteria** | | | | |
| MK91 | *Ensifer adhaerens* NBRC 100388 (NR_113893.1) | 99 | Lettuce rhizosphere | n.t. | n.t. |
| MK107 | *Ensifer adhaerens* ATCC 33212 (NR_114539.1) | 99* | Lettuce rhizosphere | n.t. | n.t. |
| MK128, **MK141** | *Ensifer adhaerens* BGRI EBC SD18-T4 (MK332555.1) | 100 | Lettuce rhizosphere, leaf | x | x |
| MK143 | *Neorhizobium galegae* 7g (CP090095.1) | 100 | Lettuce rhizosphere | n.t. | n.t. |
| MK93 | *Neorhizobium huautlense* SO2 (NR_024863.1) | 98 | Lettuce rhizosphere | n.t. | n.t. |
| MK108 | *Rhizobium giardinii* PgBE43 (MH144266.1) | 99 | Lettuce rhizosphere | n.t. | n.t. |

| | **Betaproteobacteria** | | | | |
|---|---|---|---|---|---|
| MK116 | *Acidovorax radicis* IHBB 9395 (KR085770.1) | 100 | Lettuce rhizosphere | n.t. | n.t. |
| MK115 | *Acidovorax radicis* N35 (NR_117776.1) | 100* | Lettuce rhizosphere | n.t. | n.t. |
| MK149 | *Pelomonas saccharophila* NBRC 103037 (NR_114189.1) | 99 | Lettuce rhizosphere | n.t. | n.t. |
| MK130 | *Variovorax beijingensis* T529 (MG820625.2) | 100 | Lettuce rhizosphere | 0 | n.t. |

| | **Gammaproteobacteria** | | | | |
|---|---|---|---|---|---|
| MK113 | *Pseudomonas putida* E46 (CP024086.1) | 100 | Lettuce rhizosphere | x | x |
| MK151 | *Rhizobacter dauci* H6 (NR_041585.1) | 100 | Lettuce rhizosphere | n.t. | n.t. |

| | **Actinobacteria** | | | | |
|---|---|---|---|---|---|
| MK165 | *Microbacterium yannicii* G72 (NR_117001.1) | 99 | Lettuce leaf | 0 | n.t. |
| **MK97** | *Arthrobacter pascens* DSM 20545 (NR_026191.1) | 100 | Lettuce rhizosphere, leaf | x | x |
| MK127 | *Arthrobacter pascens* JZY5-34 (MT106891.1) | 100 | Lettuce rhizosphere | n.t. | n.t. |
| MK104 | *Arthrobacter ginkgonis* SYP-A7299 (NR_156061.1) | 100 | Lettuce leaf | 0 | n.t. |
| MK118 | *Arthrobacter oryzae* XB1 (MG649992.1) | 100 | Lettuce leaf | 0 | n.t. |
| MK145 | *Arthrobacter oryzae* KV-651 (NR_041545.1) | 100 | Lettuce leaf | 0 | n.t. |
| MK137 | *Arthrobacter sulfonivorans* Asd MW-A1 (FM955888.1) | 100 | Lettuce leaf | 0 | n.t. |
| MK120, MK121 | *Pseudarthrobacter oxydans* EB38 (MH130324.1) | 100 | Lettuce leaf | 0 | n.t. |
| **MK144** | *Rhodococcus fascians* CF17 (NR_037021.1) | 100* | Lettuce leaf | x | x |
| MK163 | *Nocardioides kribbensis* KSL-2 (NR_043215.1) | 100 | Lettuce leaf | 0 | n.t. |
| MK109 | *Lentzea albidocapillata* NBRC 100372 (NR_112700.1) | 99 | Lettuce rhizosphere | n.t. | n.t. |
| MK103, MK131, MK140 | *Streptomyces subrutilus* IHBA 9229 (KR085841.1) | 100 | Lettuce rhizosphere, leaf | n.t. | n.t. |
| MK129 | *Streptomyces subrutilus* IHBA 9907 (KR085931.1) | 100 | Lettuce rhizosphere | n.t. | n.t. |
| MK126 | *Streptomyces caniscabiei* ND05-13A (MT360637.1) | 100 | Lettuce leaf | n.t. | n.t. |
| MK166 | *Streptomyces pratensis* ch24 (NR_125619.1) | 100 | Lettuce leaf | n.t. | n.t. |
| MK155 | *Streptomyces galilaeus* JCM 4757 (NR_040857.1) | 100 | Lettuce rhizosphere | n.t. | n.t. |
| MK142 | *Streptomyces gilvifuscus* T113 (NR_137389.1) | 99* | Lettuce leaf | n.t. | n.t. |
| MK157 | *Streptomyces graminifolii* JL-22 (NR_134196.1) | 100 | Lettuce leaf | n.t. | n.t. |
| MK134 | *Streptomyces venezuelae* ATCC 21018 (CP029189.1) | 100 | Lettuce rhizosphere | n.t. | n.t. |

| | **Firmicutes** | | | | |
|---|---|---|---|---|---|
| **MK 101,** MK266 | *Bacillus subtilis* DSM 10 (NR_027552.1) | 100 | Lettuce leaf | x | x |
| MK158 | *Bacillus velezensis* FZB42 (NR_075005.2) | 100 | Lettuce rhizosphere, leaf | 0 | n.t. |
| MK146 | *Domibacillus robiginosus* WS 4628 (NR_108861.1) | 99 | Lettuce leaf | 0 | n.t. |
| MK124 | *Paenibacillus* a*mylolyticus* NRRL NRS-290 (NR 025882.1) | 99* | Lettuce leaf | n.t. | n.t. |

| | **Bacteroidetes** | | | | |
|---|---|---|---|---|---|
| MK153 | *Dyadobacter ginsengisoli* Gsoil 043 (NR_041372.1) | 98 | Lettuce leaf | n.t. | n.t. |
| **MK95** | *Flavobacterium flevense* DSM 1076 (NR_114992.1) | 99* | Lettuce rhizosphere | x | x |
| MK96 | *Flavobacterium flevense* NBRC 14960 (NR_104712.1) | 99 | Lettuce rhizosphere | n.t. | n.t. |
| MK152 | *Flavobacterium sufflavum* BBQ-12 (NR_171469.1) | 99 | Lettuce rhizosphere | n.t. | n.t. |

| | | | | | |
|---|---|---|---|---|---|
| ¹Strains listed together have identical 16S rRNA gene sequences, but differ in their IGS region ²n.t. not tested; 0 no degradation activity; x degradation activity detected ^{∗}only partial 16S rRNA gene sequence available | | | | | |

**Table 1.** List of isolated strains with internal designation numbers, their closest relatives based on 16S rRNA gene analysis, their isolation source and degradation abilities.

16 strains were investigated for their capacity to degrade azoxystrobin, out of which six were found to have this capacity (see Examples below).

### Example 2. In vitro azoxystrobin degradation tests on lamb's lettuce leaves

First, the degradation capacity of selected strains was tested *in vitro* using lamb's lettuce (Valeriana-Motovilec, Samen Maier GmbH, Austria). Lamb's lettuce seeds were sown in seed trays containing a mixture (3:1 v/v) of commercial potting soil and perlite. After reaching growth stage 13 of the BBCH scale, the lettuce leaves were cut with a sterile scalpel. Round filters (Rotilabo ^{®} type 115A, 90 mm Ø, Carl Roth GmbH+Co KG, Germany) were placed in a Petri dish (90 mm Ø) and moisturised with 1 mL sterile water. Each wetted round filter was covered with 1 g lettuce leaves (top up) and 200 µL azoxystrobin stock solution per Petri dish (treatment amount was 200 mg Al/kg lettuce) were applied using an airbrush (pressure: 0.1 bar, nozzle: 0.5 mm) (Airbrush Kit with Compressor ABPST05, Timbertech, Airgoo Pneumatic BV, Netherlands). The samples were incubated at room temperature for 2 h. Then, lettuce leaves were treated with 100 µL microbial suspension (10⁶ cfu/g plant material) using an airbrush (pressure: 0.1 bar, nozzle: 0.5 mm) and incubated at room temperature overnight. All treatments were carried out in triplicates.

Sample preparation for the quantitative determination of azoxystrobin concentration was done according to Ali et al. (2015; Zaganzig J. Plant Prot. Res. 42, 1547-1553). Briefly, 1 g of cut leaves was transferred to a 5 mL tube and 1.5 mL high performance liquid chromatography (HPLC)-grade acetonitrile (Fisher Scientific, UK) containing 1% (v/v) of acetic acid was added. The sample was mixed manually for 1 min, followed by 15 s at the highest speed on the Vortex (Vortex-Genie^{™} 2, Scientific Industries SI, USA). To extract powder, 0.6 g magnesium sulfate (Sigma-Aldrich, USA) and 0.15 g anhydrous sodium acetate (Sigma-Aldrich, USA) were added, mixed manually for 1 min, vortexed for 15 s and centrifuged for 5 min at 1,503 rcf. The supernatant (0.8 mL) was transferred to a new 5 mL tube containing 120 mg magnesium sulfate and 40 mg primary and secondary amine exchange material (PSA) (Restek Corporation, USA). No graphitized carbon black (GBC) was added to the samples. The sample was shaken immediately manually for 1 min, vortexed for 30 s, and centrifuged for 5 min at 2,348 rcf. Afterwards, 0.5 mL of the upper layer were taken by syringe and needle, filtered using sterile syringe filter (PVDF Rotilabo^{®}, 0.45 µm, Carl Roth GmbH+Co KG, Germany) and stored at -20°C. Azoxystrobin concentrations were determined by HPLC. The filtered supernatant was thawed, and 1 mL of each sample was pipetted into a 2 mL screw-top glass HPLC vial (Thermo Fisher Scientific, USA).

Samples were analysed using an Agilent 1100 series HPLC with a diode array detector (DAD) and LiChrospher^{®} 100 RP-18e (5 µm) column (column length: 125 mm, pore size: 100 Å, internal diameter: 4 mm, Agilent, UK). 25µl of each sample were injected and analysed using the method of Howell et al. (2014; https://doi.org/10.1016/j.chemosphere.2013.09.048, Chemosphere 95, 370-378.); briefly: the mobile phase consisting of 75% acetonitrile (HPLC-grade, Fisher Scientific, UK) and 25% distilled water was set to a flow rate of 1.30 mL/min. Azoxystrobin concentration was determined by measuring the absorbance at 238 nm in duplicate.

Azoxystrobin recovery from the control leaf samples (samples with pesticide treatment and without bacterial treatment) was measured. Treatments showing azoxystrobin concentrations (mean of triplicates) in the range of the control were evaluated as not degraded.

The degradation potential of the seventeen selected strains and two reference strains was tested in an *in vitro* assay with lamb's lettuce leaves (Table 1). Azoxystrobin recovery from control leaf samples (samples with pesticide treatment and without bacterial treatment) was 72% of the applied concentration (theoretically 200 mg/kg; av=144 mg/kg in uninoculated control, sd=5) (Fig. 2). Most strains showed no or only low degradation of azoxystrobin after spray application on lettuce leaves. However, six strains showed degradation rates between 29% and 72%, i.e., *Flavobacterium* sp. strain MK95 (29% degradation), *Pseudomonas* sp. strain MK113 (32%), *Arthrobacter* sp. strain MK97 (56%), *Rhodococcus* sp. strain MK144 (62%), *Ensifer* sp. strain MK141 (62%) and *Bacillus* sp. strain MK101 (72%) (Fig. 2). Reference strain 1 showed 2% degradation of azoxystrobin, whereas reference strain 2 showed 47% degradation (Fig. 2).

These six isolates leading to the lowest azoxystrobin concentrations were selected for trials in the greenhouse (see Example 3).

### Example 3. Azoxystrobin degradation in greenhouse tests - foliar application

Selected strains (seven) were tested for their degradation capacity of foliar-applied fungicide using lamb's lettuce. Lamb's lettuce plants were grown as described above. At the growth stage 13 of the BBCH scale, the plants were trans-planted to 3 L pots filled with commercial potting soil (three plants per pot, four biological replicates). One day after trans-planting, 1 mL Azoxystar stock solution per pot (treatment amount 240 mg Al/kg lettuce) were applied on leaves by using an airbrush (pressure: 0.1 bar, nozzle: 0.5 mm). Subsequently, 2 mL bacterial suspension (10⁶ cfu/mL) per plant and treatment were applied two and seven days after the fungicide treatment by using an airbrush. At growth stage 17 of the BBCH scale, i.e., the seventh true leaf unfolded, the leaves were harvested and prepared for HPLC analysis. For leaf sampling, three plants of each pot were pooled into one sample and stored at -20°C. One gram of each sample was further used for quantitative analysis, as explained above. Azoxystrobin recovery from the control leaf samples (samples with pesticide treatment and without bacterial treatment) was measured. The percentage of degradation was calculated by dividing the recovered azoxystrobin amount from treated samples by the recovered azoxystrobin amount from the control samples. Recursive partitioning analysis was used for grouping the strains regarding their degradation abilities. The bacterial strains, which showed the highest azoxystrobin degradation in this trial, were selected for bacterial genome sequencing and analysis.

Results: 64% of the applied Azoxystrobin concentration were found in the control leaf samples in the plant system (153±19 mg/kg of 240 mg/kg applied). All bacterial strains showed degradation of azoxystrobin, and the best degrading strains were *Bacillus* sp. strain MK101 (>90% degradation), *Rhodococcus* sp. strain MK144 (>90%), *Pseudomonas* sp. strain MK113 (90%) and *Ensifersp.* strain MK141 (83%). *Flavobacterium* sp. strain MK95 and *Arthrobactersp.* strain MK97 showed 23% and 11% degradation, respectively. Reference strain 2 also showed high degradation of the fungicide on leaves (>90%) (Fig. 3).

### Example 4. Azoxystrobin degradation in greenhouse tests - soil application

The six degrading strains of Example 3 were assessed for their degradation the fungicide (in soil and leaves) when applied as soil inoculum.

The strains were tested for their capacity to degrade azoxystrobin when both microorganisms and fungicide were applied to the soil. A mixture (3:1 v/v) of commercial potting soil and perlite was treated with 25 mg/kg azoxystrobin (by using Azoxystar). The treated soil was equilibrated for two weeks at 4°C in the dark, before sowing the seeds. Organic lamb's lettuce seeds were surface-sterilised on a tube roller (RS-TR5, Phoenix Instrument GmbH, Germany) with 70% ethanol for 2 min followed by 0.6% sodium hypochlorite for 5 min and rinsed three times in sterile water for 1 min. Following, the seeds were imbibed with a 10⁸ cfu/mL bacterial suspension for 2 h at 28°C; for the negative control sterilised seeds were incubated in 1 x PBS buffer. At the growth stage 13 of the BBCH scale, plants were trans-planted to 3 L pots (filled with soil/perlite/azoxystrobin mixture, three plants per pot, four biological replicates). At this stage, bacteria were applied again by immersing the root system for 10 min in 10⁸ CFU/mL bacterial suspension. At the growth stage 17 of the BBCH scale, the leaves and the soil (expanded rhizosphere) were harvested and prepared for HPLC analysis. Leaf sampling and quantitative azoxystrobin determination were done as described above.

The expanded rhizospheres of the three plants per pot were mixed, and two 10 g aliquots were stored at -20°C. Sample preparation of the soil was done according to Howell et al. (2014). Briefly, 10g of soil were extracted in 50 ml conical bottom tube using 20 mL of a solution containing 75% HPLC-grade acetonitrile and 25% distilled water. The tubes were shaken for 1 h on a tube roller. Subsequently, the tubes were left 30 min to settle and centrifuged at 3,489 rcf for 2 min. Afterwards, the samples were filtered, stored, analysed and grouped as described above.

Here, 92% of the applied azoxystrobin concentration applied to soil samples was recovered in the plant system (23±2 mg/kg of 25 mg/kg applied). Azoxystrobin was translocated upward within the lettuce. In the leaves of the control plants, we measured 2% (0.48±0.01 mg/kg) of azoxystrobin that was amended to the soil. Also in this set-up, all bacterial strains showed degradation of azoxystrobin. *Rhodococcus* sp. strain MK144 and *Pseudomonas.* sp. strain MK113 showed the highest degradation of the fungicide in soils, i.e., 52% and 51% degradation, respectively, whereas *Arthrobactersp.* strain MK97 (12%), *Flavobacterium* sp. strain MK95 (14%) and *Ensifer* sp. strain MK141 (17%) showed lower degradation rates (Fig. 4A). *Bacillus* sp. strain MK101 degraded 19% of azoxystrobin in soil. The reference strain 2 showed 28% azoxystrobin degradation in soil. In this example, the best degrading strains in leaves were *Arthrobacter* sp. strain MK97 (>90% degradation) and *Pseudomonas* sp. strain MK113 (72%), whereas *Bacillus* sp. strain MK101 (39%), *Ensifersp.* strain MK141 (37%) and the reference strain 2 (38%) showed only moderate degradation (Fig. 4B). Bacteria from the genera *Rhodococcus, Flavobacterium,* and *Ensifer* have not been reported so far to degrade azoxystrobin or strobilurin fungicides.

*Arthrobacter* sp. strain MK97 was a notable degrader in the lettuce leaves when both bacteria and fungicide were applied to soil. Surface-sterilised lettuce seeds were imbibed with bacterial suspension and bacteria were applied again by immersing the root system in bacterial suspension. Due to the fact that degradation of azoxystrobin was high in leaves, we can speculate that *Arthrobacter* sp. strain MK97 might colonize the leaves predominantly from the inside of plants after seed inoculation and root immersion.

### Example 5. Whole genome sequencing

Based on their high azoxystrobin degradation activities, *Bacillus subtilis* strain MK101, *Pseudomonas kermanshahensis* strain MK113 and *Rhodococcus fascians* strain MK144 were selected for whole genome sequencing to elucidate more information on the potential mechanisms involved in the degradation.

Several genes for benzoate degradation (i.e., *catA, fadB* or *pcaC* and aminobenzoate i.e., *pdhC, ubiX* or *vanB*) may be responsible for azoxystrobin degradation by cleaving aromatic rings and were found in all three analysed genomes (Table 2). The analysis revealed that the three bacterial strains contain numerous genes predicted to be involved in pesticide degradation. Based on Prokka and eggNOG annotation in KEGG Pathways responsible for xenobiotics biodegradation and metabolism (1.11.), 561 genes potentially involved in degrading xenobiotics were found (Table 2). In the genomes of the three strains, the top seven degrading groups (out of 21 groups) were for benzoate (125 genes), aminobenzoate (63), chlorocyclohexane and chlorobenzene (40), drug metabolism - other enzymes (41), naphthalene (37), styrene (34) and caprolactam (34) degradation (bold numbers in Table 2).

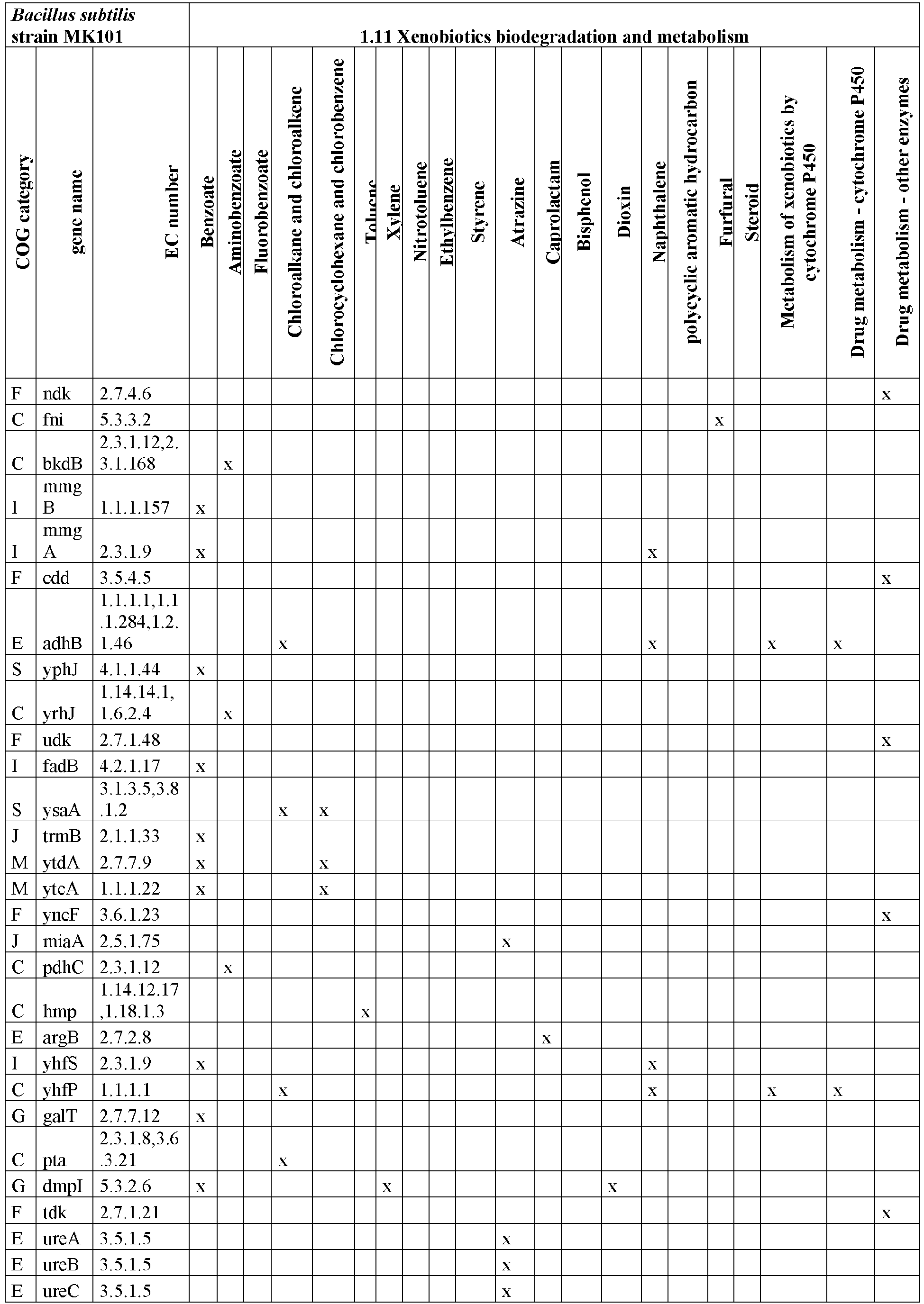

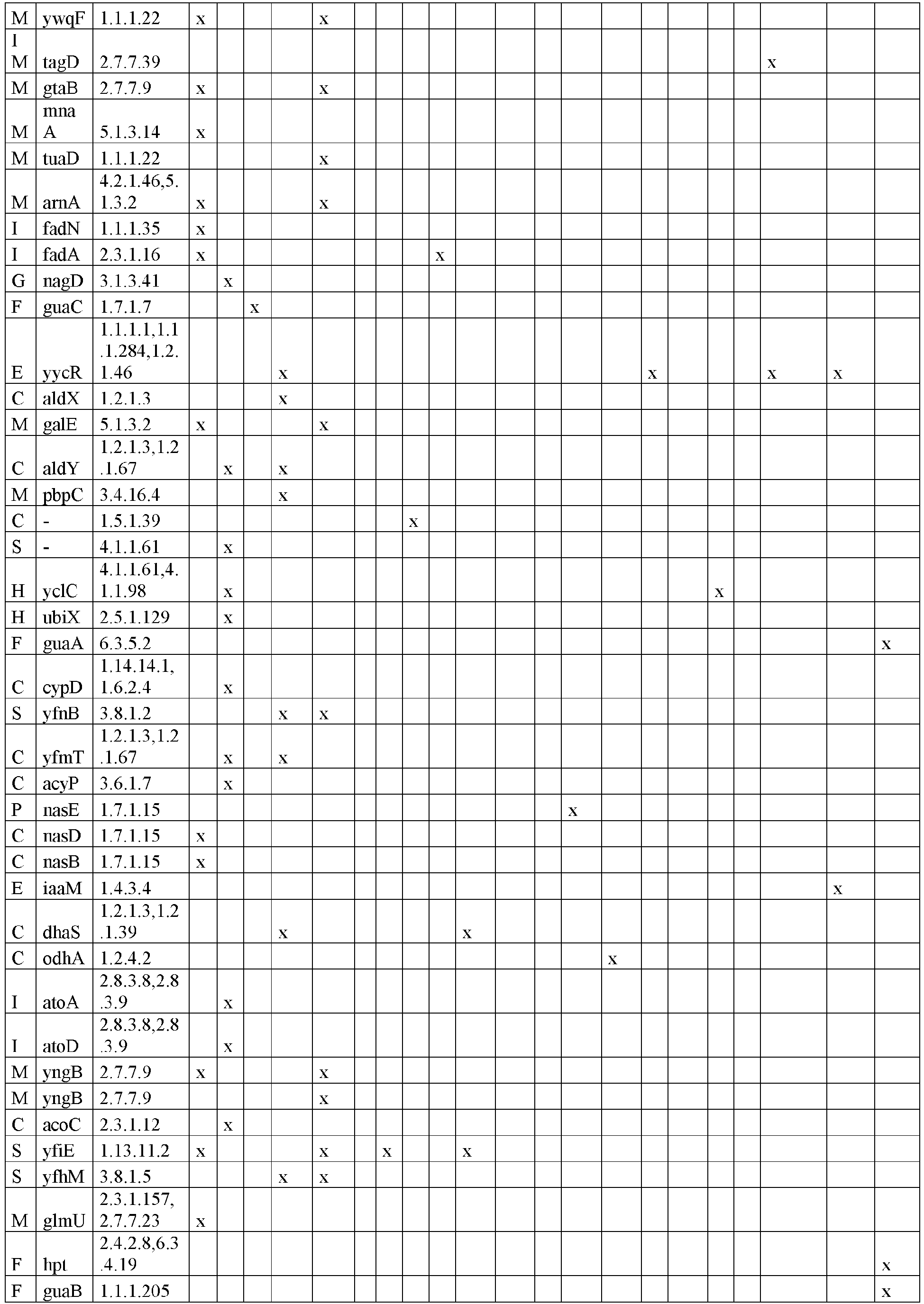

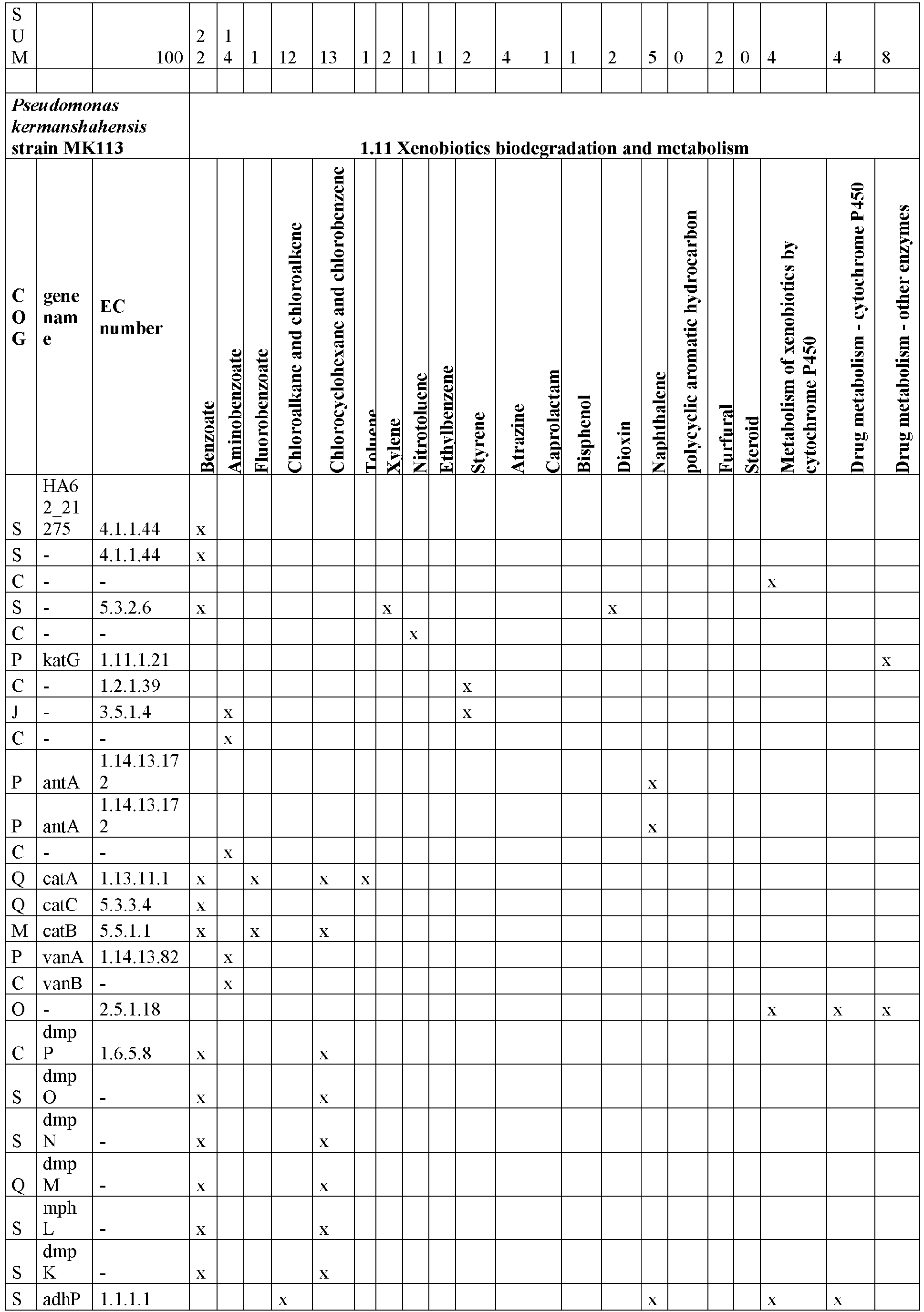

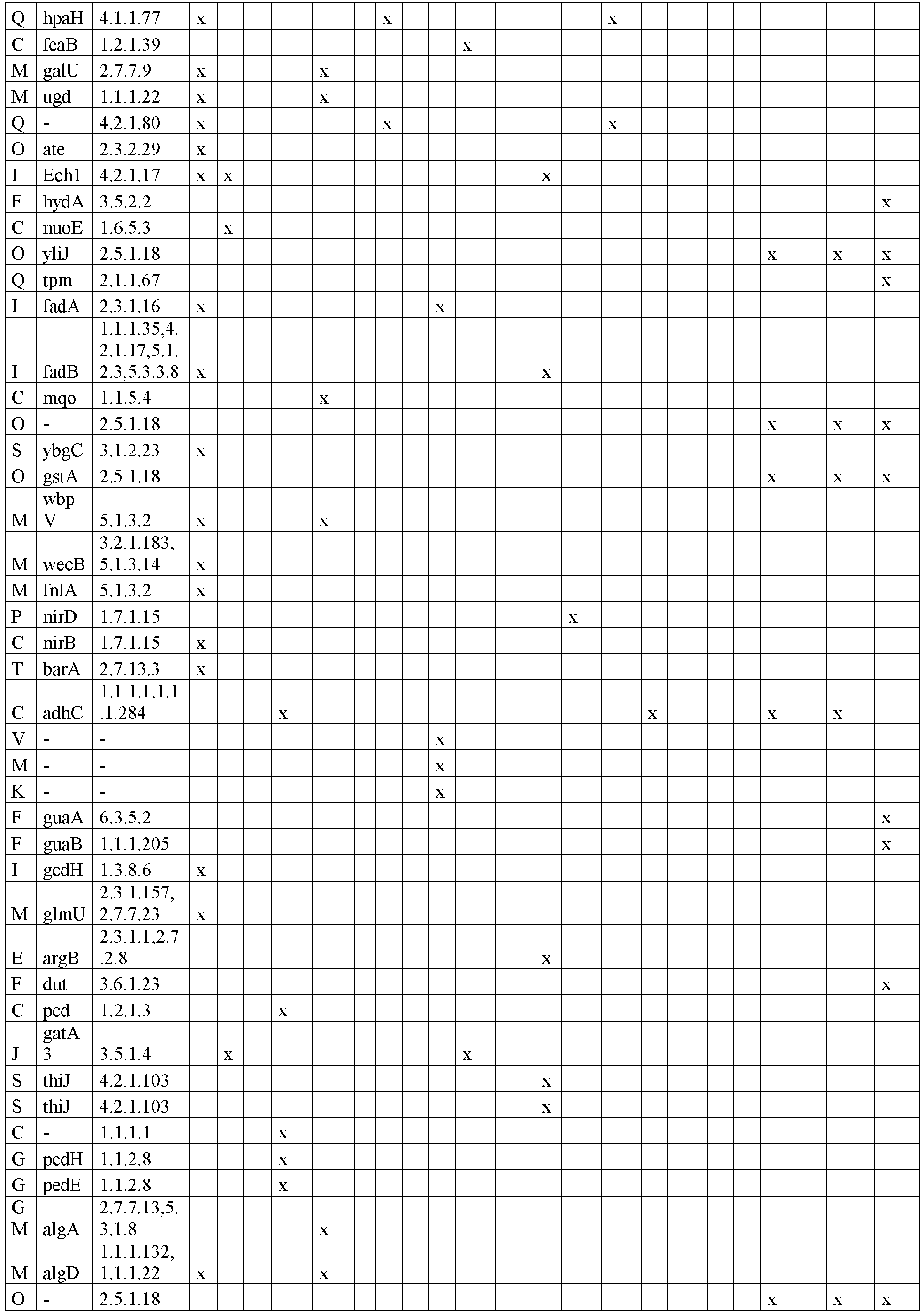

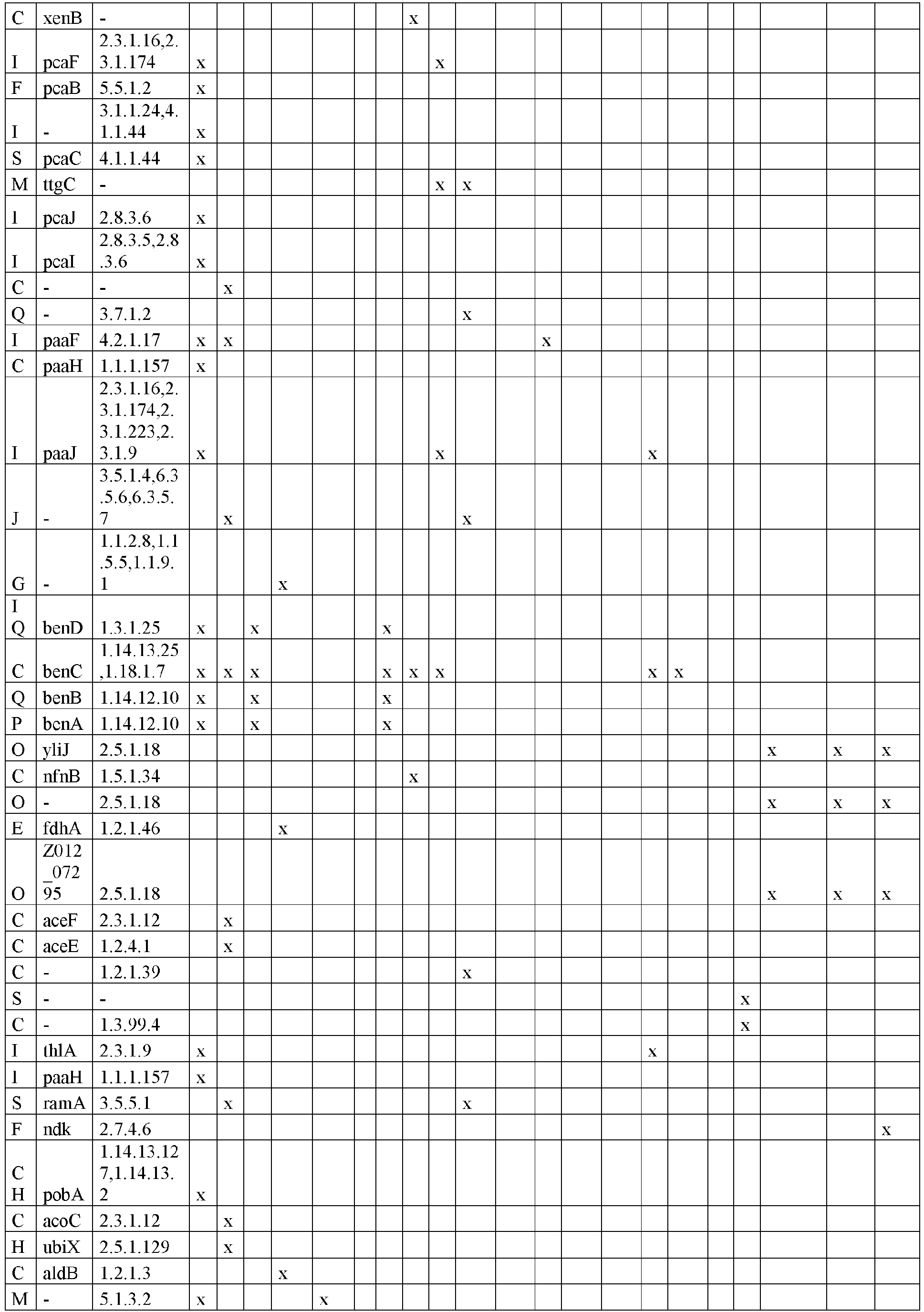

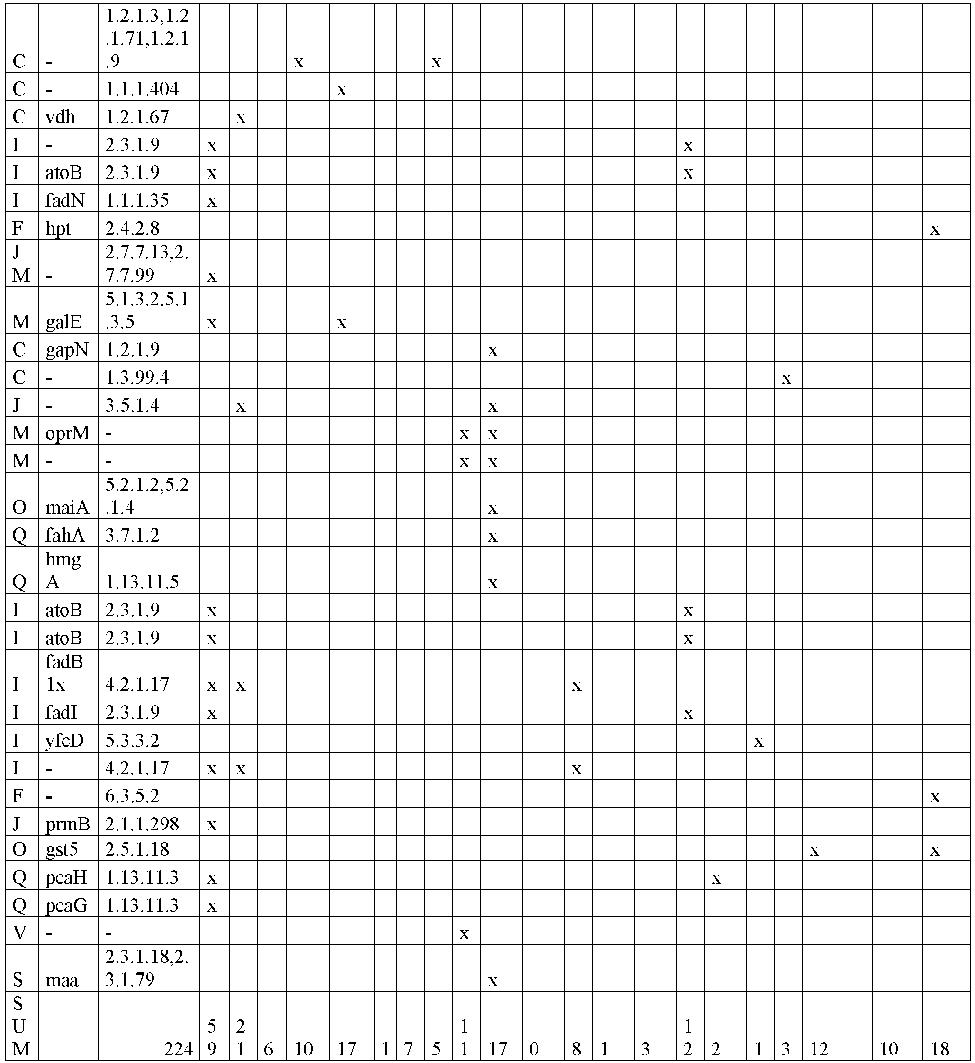

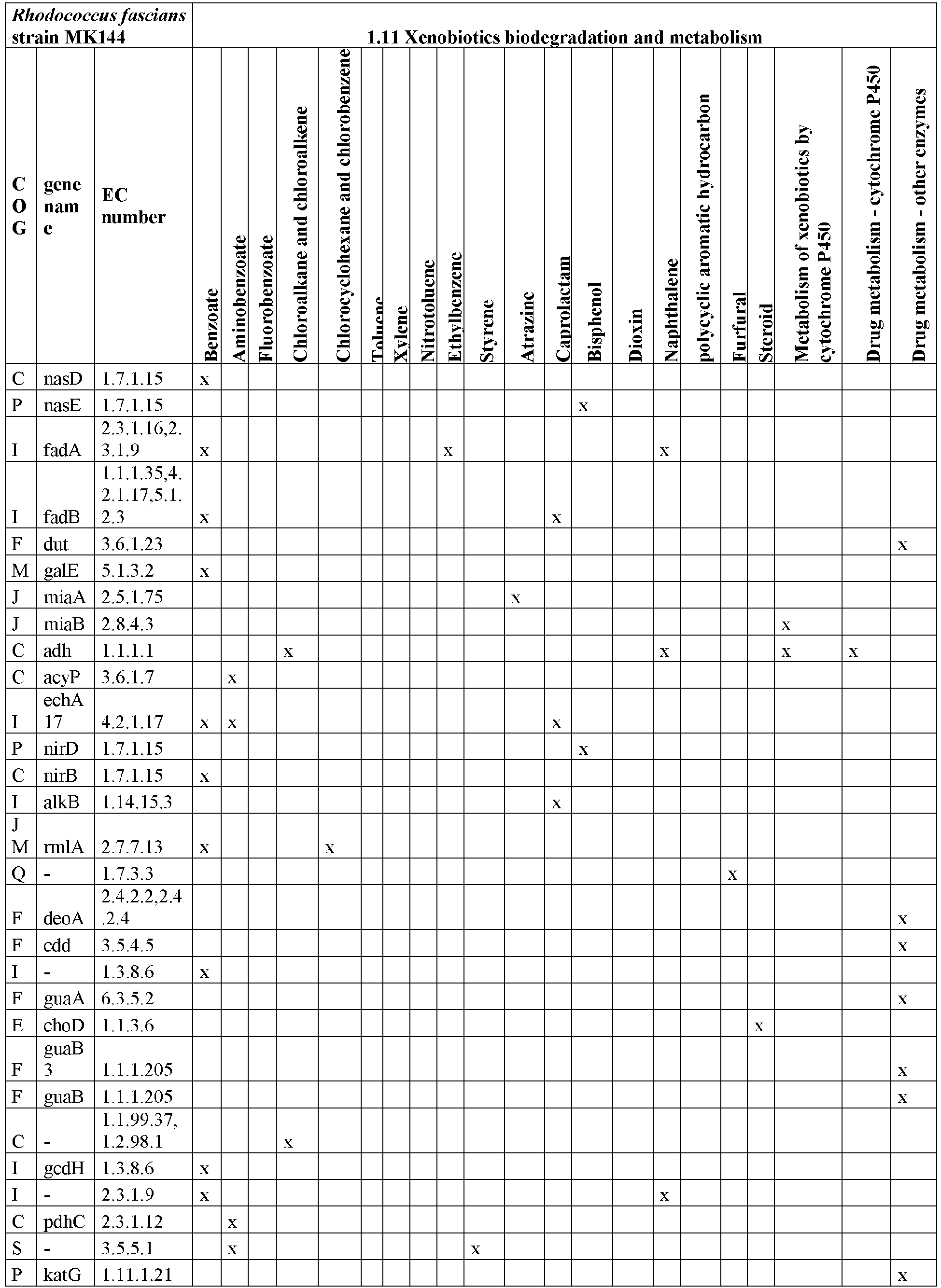

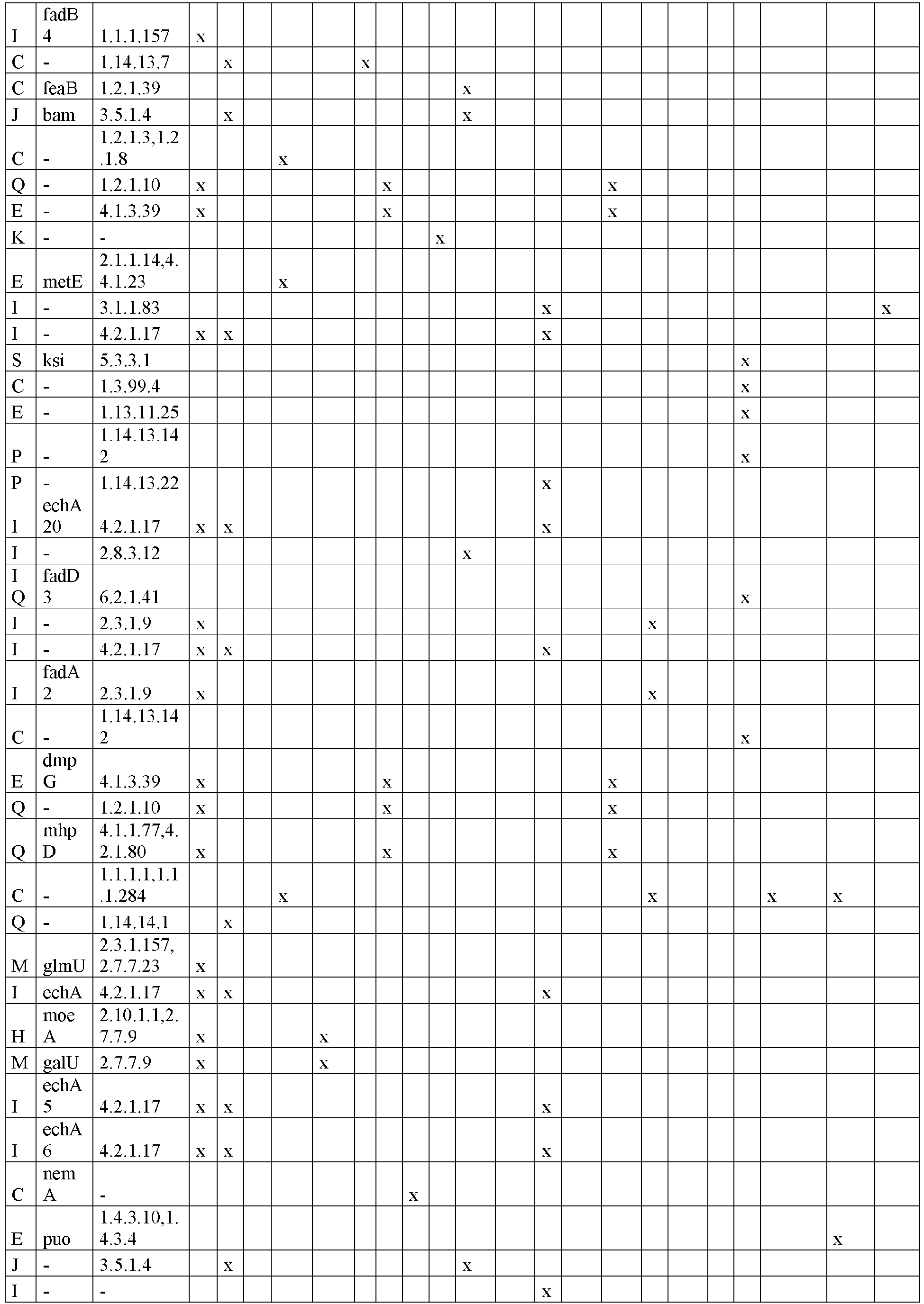

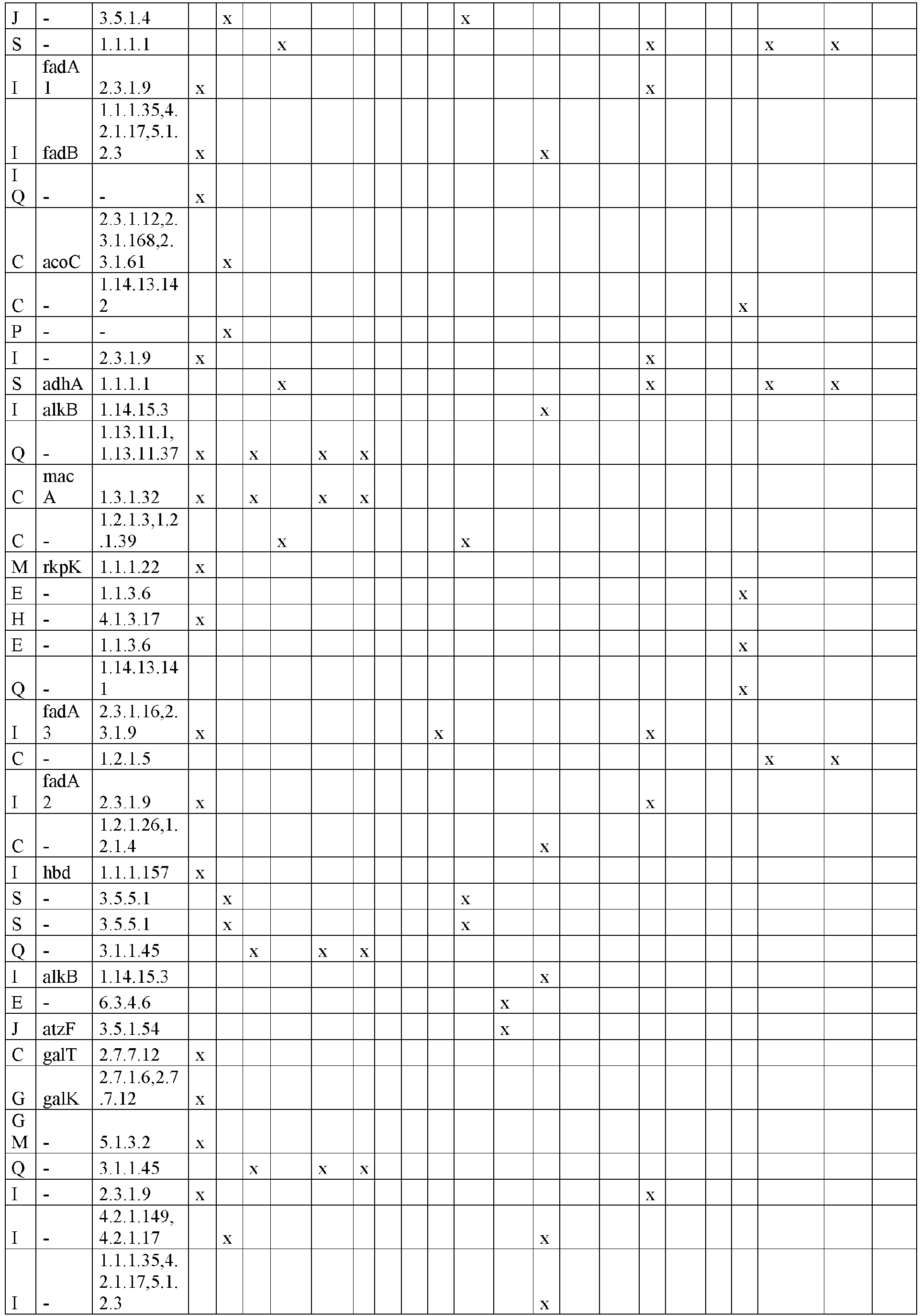

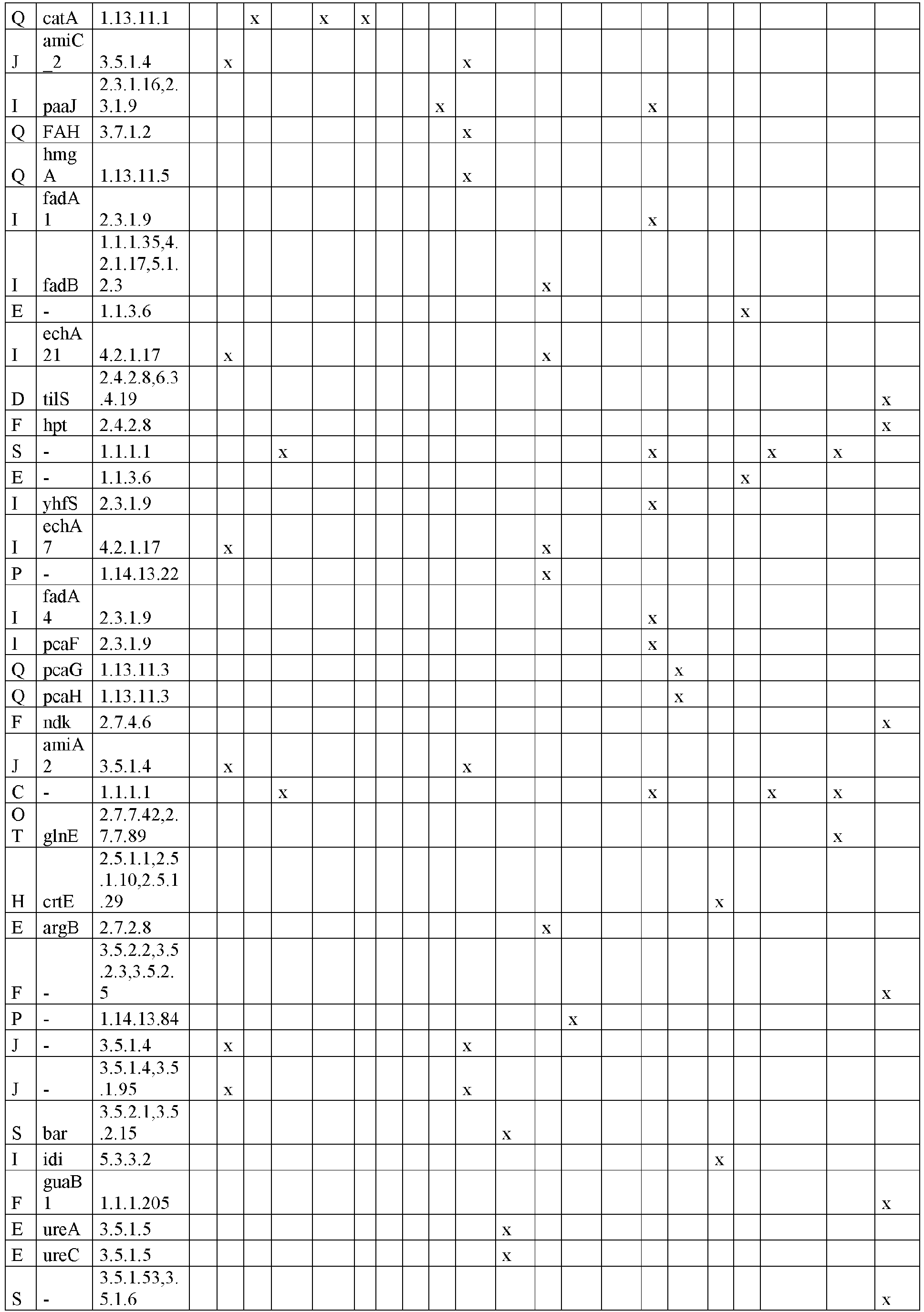

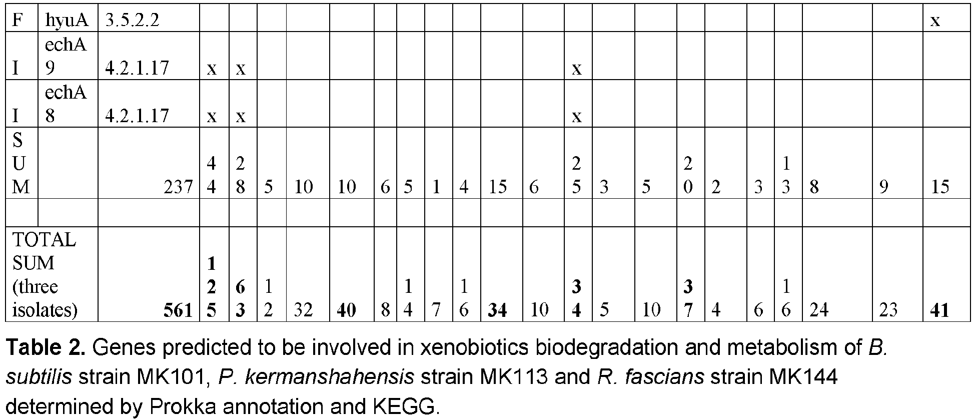

Three COG categories connected to metabolism were compared in *P*. *kermanshahensis* strain MK113, *R.* fascians strain MK144and B. *subtilis* strain MK101. *P. kermanshahensis* strain MK113 has a higher number of genes (3,072 genes) in COG category C (energy production and conversion) than *R. fascians* strain MK144 (2,129) and *B*. *subtilis* strain MK101 (1,650). In the COG categories G (carbohydrate transport and metabolism) and Q (secondary metabolites, biosynthesis, transport and catabolism) *R. fascians* strain MK144 has the highest numbers of genes (G: 1,620 and Q: 1,100). In COG group G B. *subtilis* strain MK101 has 1,592 genes and *P. kermanshahensis* strain MK113 has 1,313 genes. In *P*. *kermanshahensis* strain MK113 genome 913 genes and in *B*. *subtilis* strain MK101 608 genes were found within the COG group Q. This indicates that the genomes contain potential genes for azoxystrobin degradation. Genes coding for the enzymes in the lactoylglutathione lyase family (COG0346, COG group Q) are involved in the degradation of aromatic compounds. Nineteen genes from this COG0346 were found in the three pesticide-degrading strains, precisely 12 genes in *B*. *subtilis* strain MK101, six genes in *P*. *kermanshahensis* strain MK113 and one gene in *R. fascians* strain MK144 genome (Table 3).

Furthermore, genes coding for the enzymes in the lactoylglutathione lyase family are known to be involved in the degradation of aromatic compounds by employing dioxygenase enzymes to activate and cleave the aromatic ring (Mesarch et al., 2000; Appl. Environ. Microbiol. 66, 678-683. https://doi.org/10.1128/AEM.66.2.678-683.2000). Genes encoding the enzymes in the lactoylglutathione lyase family (COG0346) (Elufisan et al., 2020; PeerJ 2020. https://doi.org/10.7717/peerj.8102) were found in all three sequenced bacterial genomes, particularly in the *B*. *subtilis* strain MK101 genome (COG0346; Table 3).

**Table 3. Genes of B. subtilis strain MK101, P. kermanshahensis strain MK113 and R. fascians strain MK144 which code for enzymes in the lactoylglutathione lyase family (COG0346, COG group Q). They help the degradation of aromatic compounds.**

| **Strain** | | **Description** | **Gene name** | **EC number** |
|---|---|---|---|---|
| | MK101 | Glyoxalase/Bleomycin resistance protein/Dioxygenase superfamily | yqjT | - |
| | MK101 | Lactoylglutathione lyase and related lyases | mce | 5.1.99.1 |
| | MK101 | Lactoylglutathione lyase and related lyases | ykcA | - |
| | MK101 | Glyoxalase/Bleomycin resistance protein/Dioxygenase superfamily | ywkD | - |
| | MK101 | Glyoxalase/Bleomycin resistance protein/Dioxygenase superfamily | yurT | - |
| | MK101 | Glyoxalase/Bleomycin resistance protein/Dioxygenase superfamily | yyaH | 4.4.1.5 |
| | MK101 | Glyoxalase/Bleomycin resistance protein/Dioxygenase superfamily | cadI | 4.4.1.5 |
| | MK101 | Glyoxalase/Bleomycin resistance protein/Dioxygenase superfamily | yycE | - |
| | MK101 | Glyoxalase | ywbC | 4.4.1.5 |
| | MK101 | Lactoylglutathione lyase and related lyases | ydfO | - |
| | MK101 | Glyoxalase/Bleomycin resistance protein/Dioxygenase superfamily | yetH | - |
| | MK101 | Lactoylglutathione lyase and related lyases | yodE | - |
| | MK113 | Glyoxalase bleomycin resistance protein dioxygenase | - | - |
| | MK113 | Glyoxalase/Bleomycin resistance protein/Dioxygenase superfamily | - | - |
| | MK113 | Bleomycin resistance protein | - | - |
| | MK113 | Glyoxalase bleomycin resistance protein dioxygenase | - | - |
| | MK113 | Glyoxalase bleomycin resistance protein dioxygenase | - | - |
| | MK113 | Catalyzes the conversion of hemimercaptal, formed from methylglyoxal and glutathione, to S-lactoylglutathione | gloA | 4.4.1.5 |
| | MK144 | Glyoxalase/Bleomycin resistance protein/Dioxygenase superfamily | gloA_2 | - |

After removing signal peptidases, Clp, Lex and Lon proteases, 14 subtilase-like serine proteases (E.C. 3.4.21.-) were found in the genome of *B*. *subtilis* strain MK101, no subtilase-like serine proteases were found in *R. fascians* strain MK144 and *P*. *kermanshahensis* strain MK113 (Table 4).

**Table 4. Subtilase-like serine proteases from species with strobilurin methylesterase activity. The genomes of B. subtilis strain MK101, P. kermanshahensis strain MK113 and R. fascians strain MK144 were interrogated for subtilisin-like enzymes by E.C. designation (E.C. 3.4.21.-). Proteases with specific cellular functions (signal peptidases, Clp, Lex and Lon proteases) have been excluded from the list.**

| **Strain** | | **COG** | **Description** | **Gene name** | **EC number** | **PFAMs** |
|---|---|---|---|---|---|---|
| | MK101 | U | Belongs to the peptidase S26 family | lepB | 3.4.21.89 | Peptidase_S24 |
| | MK101 | M | Stage IV sporulation protein B | spoIVB | 3.4.21.116 | PDZ,PDZ_2,Peptidase_S55 |
| | MK101 | O | membrane protein (homolog of Drosophila rhomboid) | gluP | 3.4.21.105 | Rhomboid,TPR_2,TPR_8 |
| | MK101 | U | Belongs to the peptidase S26 family | sipT | 3.4.21.89 | Peptidase_S24 |
| | MK101 | O | COG0265 Trypsin-like serine proteases, typically periplasmic, contain C-terminal PDZ domain | htrA | 3.4.21.107 | PDZ_2,Trypsin_2 |
| | MK101 | U | Belongs to the peptidase S26 family | spsB | 3.4.21.89 | Peptidase_S24 |
| | MK101 | O | Belongs to the peptidase S8 family | aprE | 3.4.21.62 | Inhibitor_I9,Peptidase_S8,SLH |
| | MK101 | O | Belongs to the peptidase S8 family | prtS | 3.4.21.110,3. 4.21.96 | FIVAR,FlgD_ig,Gram_pos_anchor,I nhibitor_I9,PA,Peptidase_S8,YSIRK _signal,fn3_5 |
| | MK101 | M | Belongs to the peptidase S41A family | ctpB | 3.4.21.102 | PDZ,PDZ_2,PG_binding_1,Peptidas e_S41 |
| | MK101 | O | COG0265 Trypsin-like serine proteases, typically periplasmic, contain C-terminal PDZ domain | htrA | 3.4.21.107 | PDZ_2,Trypsin_2 |
| | MK101 | O | COG0265 Trypsin-like serine proteases, typically periplasmic, contain C-terminal PDZ domain | yyxA | 3.4.21.107 | PDZ_2,Trypsin_2 |
| | MK101 | O | Belongs to the peptidase S8 family | epr | 3.4.21.62 | Peptidase_S8,SLH,fn3 |
| | MK101 | U | Belongs to the peptidase S26 family | lepB | 3.4.21.89 | Peptidase_S24 |
| | MK101 | M | Belongs to the peptidase S41A family | ctpA | 3.4.21.102 | PDZ,PDZ_2,PG_binding_1,Peptidas e, S41 |

Besides their capacity to degrade azoxystrobin, their capability to increase plant growth was analysed. Therefore, genes known to be involved in plant growth promotion were analysed, and were found in the genomes of all three strains. *B. subtilis* strain MK101, *P. kermanshahensis* strain MK113 and *R. fascians* strain MK144 host genes responsible for the production of indole-3-acetic acid (IAA), the major naturally occurring auxin.

Auxins are frequently found in plant-associated bacteria and play a central role in plant growth and plant body development (Mitter et al., 2013; Adv. Agron. 121, 381-445. https://doi.org/10.1016/B978-0-12-407685-3.00007-4). Furthermore, *P. kermanshahensis* strain MK113 has genes encoding TonB-dependent iron receptors, which can promote plant growth indirect by the transport of ferric-siderophore complexes (Mitter et al., 2013). According to its genome, this strain is also able to produce 1-aminocyclopropane-1-carboxylate (ACC) deaminase, which degrades ACC, the precursor of ethylene, in plants to 2-oxobutyrate and ammonia. ACC deaminase reduces growth-retarding effects of ethylene produced by plants in response to biotic and abiotic stress (Glick, 2005; FEMS Microbiol. Lett. 251, 1-7. https://doi.org/10.1016/j.femsle.2005.07.030). Quorum sensing is involved in the regulation of many microbial activities, and particularly N-acyl-homoserine lactones (AHL) are key regulating molecules in quorum sensing (Hartmann et al., 2021; Pathogens 10. https://doi.org/10.3390/pathogens10121561) in Proteobacteria. AHLs produced by soil bacteria have also been found to induce a plant response, such as resistance towards pathogens (Liu et al., 2020, BMC Plant Biol. 20, 1-13. https://doi.org/10.1186/s12870-019-2228-6; Schikora et al., 2016, Plant Mol. Biol. 90,605-612. https://doi.org/10.1007/s11103-016-0457-8). In this study, the *P. kermanshahensis* strain MK113 genome suggests the production of AHLs. In addition, *B*. *subtilis* strain MK101 and *R. fascians* strain MK144 possess genes responsible for IAA production, the genome of *R. fascians* strain MK144 also suggest ACC deaminase activity in this strain. These results indicate that all three strains have the potential to enhance the stress resilience and improve the growth of plants.

In the last step, genes related to plant-microbe interactions were analyzed. The analysis showed that the strains do not have genes responsible for siderophore production, however *P*. *kermanshahensis* strain MK113 has genes encoding TonB-denpendent iron receptors (*bfrG, btuB, cirA, exbB, fcuA, fecA, fepA, fiuA, foxA, fpvA, iutA, oprC, phuR, ronB2, sftP, tonB, viuA* and *yncD*). In the genomes of *B*. *subtilis* strain MK101, *P. kermanshahensis* strain MK113 and *R*. *fascians* strain MK144 genes for IAA synthesis pathway (*trpA* and *trpB*) were found. Concerning quorum sensing-related genes, *B*. *subtilis* strain MK101 and *P. kermanshahensis* strain MK113 showed related genes in their genomes. Precisely, autoinducer-2 system was identified in 8. *subtilis* strain MK101 genome, whereas the AHL-based systems were identified in *P*. *kermanshahensis* strain MK113. The gene *acdS* was found in the genomes of *P*. *kermanshahensis* strain MK113 and *R. fascians* strain MK144, but not in 8. *subtilis* strain MK101 (Table 5).

**Table 5. Genes related to plant-microbe interactions. The genomes of B. subtilis strain MK101, P. kermanshahensis strain MK113 and R. fascians strain MK144 were interrogated for ferric-siderophore complexes, Indole acetic acid (IAA) synthesis pathways, quorum sensing and 1-aminocyclopropane-1-carboxylic acid (ACC) deaminase.**

| **Strain** | **Ferric-siderophore complexes** | **IAA synthesis pathway** | **Quorum sensing** | **ACC deaminase** |
|---|---|---|---|---|
| MK101 | | x | x | |
| MK113 | | x | x | x |
| MK144 | | x | | x |

In summary, we conclude that the rhizosphere and the leaves of lettuce plants grown on fungicide contaminated soils are a reservoir for bacteria that can degrade active fungicide ingredients. Three strains, *B*. *subtilis* strain MK101, *P. kermanshahensis* strain MK113 and *R. fascians* strain MK144 were found to be efficient azoxystrobin degraders and their genomes suggest multiple pathways potentially involved in degradation and plant growth promotion.

### Example 6. Microbial compositions for foliar application

As described, at least one bacterium or the combination of bacteria can be applied with at least one additive in form of a composition, and preferably, the composition can be applied as a foliar. Foliar spray applications are well known in the art (Preininger et al. Applied Microbiology and Biotechnology (2018) 102:7265-7282; herein incorporated by reference as far as relating to the manufacture of spray applications of microorganisms).

For foliar spray application, we recommend mixing 1.5 vol% up to 2.5 vol% of the bacterial solution/s with a cfu/ml of between 1 × 10⁷ and 1 × 10¹⁰ with water. The amount of water varies with the plant size. Larger plants (or plants with a higher number of leaves) require more water than smaller ones. Thus, with a constant concentration of bacteria in the spray solution, the amount of bacteria applied increases with the size of the plant or with the number of leaves. In order to achieve further positive effects, a combination with biostimulants or biological plant strengtheners such as products containing plant extracts, other microorganisms like plant growth-promoting bacteria or humic substances in an amount of 0.1 vol% up to 0.5 vol% is possible.

When spraying the foliar composition on the leaves, it is important to spray directly on the aboveground parts of the plant, to consider the interaction with the plant leaf surface. This is important for the viability of the bacteria. Further factors like plant stage, plant environment, spray frequency or droplet size and velocity can have an impact on microbial sprays.

A potential composition could be to use 1 vol% of the *Bacillus subtilis* MK101 solution with a cfu/ml of between 1 × 10⁷ and 1 × 10¹⁰, 1 vol% of the *Rhodococcus fascians* MK144 solution with a cfu/ml of between 1 × 10⁷ and 1 × 10¹⁰ and 98% of water for spray application.

Another potential composition could be to use 0.5 vol% of the *Bacillus subtilis* MK101 solution with a cfu/ml of between 1 × 10⁷ and 1 × 10¹⁰, 0.5 vol% of the *Rhodococcus fascians* MK144 solution with a cfu/ml of between 1 × 10⁷ and 1 × 10¹⁰, 0.5% of the *Pseudomonas kermanshahensis* MK113 solution with a cfu/ml of between 1 × 10⁷ and 1 × 10¹⁰ and 98.5% of water for spray application.

## Claims

1. A method for reducing the content of a strobilurin or a strobilurin derivative on a plant surface, in a plant and/or in soil, comprising
(i) applying at least one bacterium selected from the group consisting of *Bacillus subtilis* MK101 with the accession no. DSM34510, *Rhodococcus fascians* MK144 with the accession no. DSM34512, *and Pseudomonas kermanshahensis* MK113 with the accession no. DSM34511, or a bacterium with an 16S rRNA sequence having at least 97%, 98%, or 99% sequence identity with the 16S rRNA sequence of *Bacillus subtilis* MK101 of SEQ ID NO:1, *Rhodococcus fascians* MK144 of SEQ ID NO:2, or *Pseudomonas kermanshahensis* MK113 of SEQ ID NO:3, or any combination of these bacteria, to at least one aerial part of the plant and/or to the soil, and
(ii) incubating the plant and/or the soil for a time sufficient to reduce the content of the strobilurin on the plant surface and/or in the plant and/or in the soil.

2. The method according to claim 1, wherein the at least one bacterium or the combination of any of these bacteria is applied alone, or in conjunction with other microorganisms.

3. The method according to claim 1 or 2, wherein the at least one bacterium or the combination of bacteria is applied in form of a composition, wherein the composition can be applied as a foliar.

4. The method according to any one of claims 1-3, wherein the concentration of the at least one bacterium applied is from about 1 × 10⁵ to about 1 × 10⁷ cfu per plant per application, or from about 5 × 10⁵ to about 5 × 10⁷ cfu per m² of soil per application.

5. Use of at least one bacterium selected from the group consisting of *Bacillus subtilis* MK101 with the accession no. DSM34510, *Rhodococcus fascians* MK144 with the accession no. DSM34512, and *Pseudomonas kermanshahensis* MK113 with the accession no. DSM34511, or a bacterium with an 16S rRNA sequence having at least 97%, 98%, or 99% sequence identity with the 16S rRNA sequence of *Bacillus subtilis* MK101 of SEQ ID NO:1, *Rhodococcus fascians* MK144 of SEQ ID NO:2, or *Pseudomonas kermanshahensis* MK113 of SEQ ID NO:3, or any combination of these bacteria, for reducing the strobilurin content in a plant, on a plant surface and/or in soil, by applying said at least one bacterium or combination of bacteria to at least one aerial part of the plant and/or to the soil.

6. The use according to claim 5, wherein the at least one bacterium or the combination of any of these bacteria is applied alone, or in conjunction with other microorganisms.

7. The use according to claim 5 or 6, wherein the at least one bacterium or the combination of bacteria is applied in form of a composition, wherein the composition can be applied as a foliar.

8. The method according to any one of claims 1-4 or the use according to any one of claims 5-7, wherein the at least one bacterium or the combination of bacteria or the composition are applied to the leaves of the plant, to the rhizosphere of the plant, and/or to the soil.

9. The method or the use according to claim 8, wherein the at least one bacterium or the combination of bacteria or the composition are applied to the leaves of the plant.

10. The method or use according to any one of claims 1-9, wherein the strobilurin is selected from the group consisting of Azoxystrobin, Dimoxystrobin, Fluoxastrobin, Kresoxim-methyl, Metominostrobin, Orysastrobin, Picoxystrobin, Pyraclostrobin, and Trifloxystrobin, preferably the strobilurin is Azoxystrobin.

11. A composition comprising (a) at least one bacterium selected from the group consisting of *Bacillus subtilis* MK101 with the accession no. DSM34510, *Rhodococcus fascians* MK144 with the accession no. DSM34512, and *Pseudomonas kermanshahensis* MK113 with the accession no. DSM34511, or a bacterium with an 16S rRNA sequence having at least 97%, 98%, or 99% sequence identity with the 16S rRNA sequence of *Bacillus subtilis* MK101 of SEQ ID NO:1, *Rhodococcus fascians* MK144 of SEQ ID NO:2, or *Pseudomonas kermanshahensis* MK113 of SEQ ID NO:3, or any combination of these bacteria, optionally in further combination with other microorganisms, and (b) at least one additive.

12. The composition according to claim 11, wherein the composition is a foliar.

13. The method, use or composition according to any one of the preceding claims, wherein the at least one bacterium does not express *strH,* optionally GenBank Acc No. MT992707 and/or, wherein the at least one bacterium has genes for (i) benzoate degradation, preferably *catA, fadB* and/or *pcaC*; and/or (ii) aminobenzoate degradation, preferably *pdhC, ubiX* and/or *vanB;* and/or (iii) genes encoding enzymes in the lactoylglutathione lyase family, preferably *cadI, gloA, gloA_2, mce, ydfO, yetH, ykcA, yodE, yqjT, yurT, ywbC, ywkD, yyaH* and/or *yycE.*

14. A bacterium selected from *Bacillus subtilis* MK101 with the accession no. DSM34510, *Rhodococcus fascians* MK144 with the accession no. DSM34512, *Pseudomonas kermanshahensis* MK113 with the accession no. DSM34511, or a derivative strain of any of these.

15. The bacterium of claim 14, wherein the derivative strain is a bacterium of *Bacillus subtilis* MK101 with the accession no. DSM34510, *Rhodococcus fascians* MK144 with the accession no. DSM34512, or *Pseudomonas kermanshahensis* MK113 with the accession no. DSM34511 that has been genetically modified or contains a nucleic acid that is heterologous to the strain, or contains an additional copy of a endogenous nucleic acid, or has a endogenous rearrangement of genes relative to the deposited strain.
